# EUROPÄISCHE PATENTSCHRIFT

(11) **EP 0 369 182 B1**
(45) Veröffentlichungstag und Bekanntmachung des Hinweises auf die Patenterteilung: **29.03.1995**
(21) Anmeldenummer: 89119408.6
(22) Anmeldetag: 19.10.1989
(51) Int. Cl.: C07H 11/04, A61K 31/70

(54) **Tumorhemmende Saccharid-Konjugate**
Antitumour saccharide conjugates
Conjugués de saccharides comme agents anti-tumeurs

(30) Priorität: 20.10.1988 DE 3835772
(43) Veröffentlichungstag der Anmeldung: 23.05.1990
(73) Patentinhaber: Deutsches Krebsforschungszentrum Stiftung des öffentlichen Rechts, 69120 Heidelberg (DE)
(72) Erfinder: Wiessler, Manfred, Prof.Dr., D-6900 Heidelberg (DE); Dickes, Michael, D-6901 Dossenheim (DE)
(74) Vertreter: Müller-Boré & Partner Patentanwälte

(56) Entgegenhaltungen:
- EP-A- 0 098 601
- DD-A- 122 386
- CHEMICAL ABSTRACTS, Band 81, Nr. 5, 5. August 1974, Seite 454, ZusammenfassungNr. 25895f, Columbus, Ohio, US; V.M. OVRUTSKII: "2,3,4,6-Tetra-O-acetyl-alpha-D-glucopyranosyl esters of N-aryl-N',N'-bis(2-chloroethyl)diamidophosphoricacid", & ZH. OBSHCH. KHIM. 1974, 44(4), 896-9
- CHEMICAL ABSTRACTS, Band 69, Nr. 19, 4. November 1968, Seite 7279,Zusammenfassung Nr. 77679k, Columbus, Ohio, US; I.P. GUDKOVA et al.: "Glycosylphosphites and glycosyl phosphonites", & ZH. OBSHCH. KHIM.1968, 38(6), 1340-4

## Beschreibung

Krebserkrankungen stehen in der Bundesrepublik Deutschland nach den Herz-Kreislaufkrankheiten an zweiter Stelle in der Mortalitätsstatistik. Zu den etablierten Therapien der Krebserkankungen zählen heute neben der Operation und der Bestrahlung auch die antineoplastische Chemotherapie.

Trotz vorzüglicher Operationstechnik, verbesserter Strahlentherapie und vielen neu entwickelten Chemotherapeutika ist es letztendlich nicht gelungen, die Heilungsrate maligner Tumore in den letzten Jahren zu verbessern, wenn auch bei einzelnen Tumorarten, wie z.B. beim Morbus Hodgkin, sehr gute Ergebnisse erzielt wurden.

Es besteht also noch das Bedürfnis, auch in der Chemotherapie grundlegende Verbesserungen, basierend auf ständig anwachsenden Kenntnissen über die Biochemie der Tumorzelle, zu ermöglichen.

Das Hauptziel bei der Neuentwicklung antineoplastisch wirkender Chemotherapeutika ist die Verbesserung der Selektivität und somit eine Verringerung unerwünschter Nebenwirkungen.

Zwar kennt man mittlerweile viele biochemische Unterschiede zwischen Tumorzelle und normaler Zelle, doch sind diese Unterschiede nicht allzu signifikant.

Eine Vielzahl der heute eingesetzten Substanzen verfügt daher bereits über eine gewisse Selektivität und somit über einen brauchbaren therapeutischen Index, jedoch ist man von einer absoluten Selektivität noch weit entfernt.

Eine Möglichkeit, dieses Ziel zu erreichen, ist die Anwendung von "Prodrugs", d.h. Arzneistoffen, die an oder in der Zielzelle besonders aktiviert werden oder aber von Nicht-Zielzellen besonders stark detoxifiziert werden. Gemäß einem anderen Ansatz versucht man, den Arzneistoff an oder in die Zielzelle zu schleusen oder zumindest dort anzureichern ("Drug Targeting").

Viele Konzepte des Drug Targeting basieren auf einer spezifischen Bindung eines Pharmakons an die Zielzelle oder auf unterschiedlichen Aufnahmemechanismen zwischen Nicht-Zielzelle und Zielzelle. Auch quantitative Unterschiede können hierbei ausgenutzt werden.

So ist man z.B. mit Hilfe der Hybridisierungstechnik (Köhler und Milstein, 1975, Nature 256, 495) in der Lage, gezielt monoklonale Antikörper (MAB's) herzustellen und mit deren Hilfe tumorassoziierte Antigene (TAA's) zu erkennen.

Die herzustellenden Glycosidester sind nach an sich bekannten Methoden, insbesondere durch die weiter entwickelte Koenigs-Knorr-Reaktion oder die Imidatmethode zugänglich.

Eine Zusammenfassung für diese Methoden sowie für die stereoselektive Glycosylierung, für die heute je nach gewünschter Stereochemie der Verknüpfung, drei grundlegende Methoden zur Verfügung stehen, findet sich insbesondere in Paulsen, 1984, Chem. Soc. Rev. 13, 15.

Es ist jedoch bekannt, daß trotz der vielen zur Verfügung stehenden Glycosylierungsmethoden nicht jede gewünschte Verknüpfung steroselektiv in zufriedenstellenden Ausbeuten darstellbar ist. Jeder Glycosyltransfer stellt ein eigenständiges Problem dar und universelle Reaktionsbedingungen sind oftmals nicht gegeben (Schmidt 1986, Angew. Chem. 98, 213).

In CA 81:25895 f werden 2,3,4,6-Tetra-O-acetyl-α-D-glucopyranosylester von N-Aryl-N',N'-bis(2-chlorethyl)diaminophosphorsäure sowie ein Verfahren zu deren Herstellung beschrieben.

Demgemäß betrifft die Erfindung Glycokonjugate bestimmter wirksam angreifender Antitumormittel zur Verwendung als antineoplastische Mittel unter weitgehendem Erhalt ihrer Aktivität, jedoch unter starker Verminderung ihrer Toxizität.

Als typische Beispiele wurden in der beschriebenen Weise Konjugate der folgenden allgemeinen Formel dargestellt. 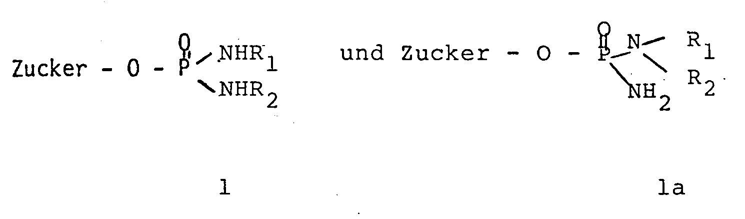
wobei die Verknüpfung des Zuckers mit der Phosphoramid-Lost-Funktion vorzugsweise in der 1-Stellung erfolgt. Als wichtigste Zucker sind zu nennen:
Galactose, Mannose, Glucose, Mannan, Galactan, Glucan sowie auch verzweigte Zucker, insbesondere in 3- und 6-Position, wobei jedoch grundsätzlich alle Zucker, also Mono-, Di- oder Polysaccharide in allen exisitierenden isomeren und enantiomeren Formen, einsetzbar sind.

R₁ und R₂ können gleich oder verschieden sein und folgende Bedeutungen haben:
Wasserstoff, niedriges C₁ -C₄-Alkyl, C₁-C₆ Halogenalkyl, vorzugsweise C₁-C₄-Halogenalkyl und insbesondere C₂Halogenalkyl.

Als Schutzgruppen können alle für OH-Funktionen allgemein üblichen Schutzgruppen verwendet, z. B. Benzyl-, Acetyl-, Tritylgruppen und die Abspaltung erfolgt in ebenfalls an sich bekannter Weise, enzymatisch, hydrogenolytisch, sauer oder alkalisch.

Ganz allgemein können die erfindungsgemäß gesuchten und eingesetzten glycosylisch gekoppelten Phosphamid mustard und Ifosfamid mustard folgendermaßen dargestellt werden:
Die zu koppelnden Einheiten, die Phosphorverbindungen können nach der Arbeitsweise von Lorenz und Wießler, 1985, Arch. Pharm. 318, 577, hergestellt werden.

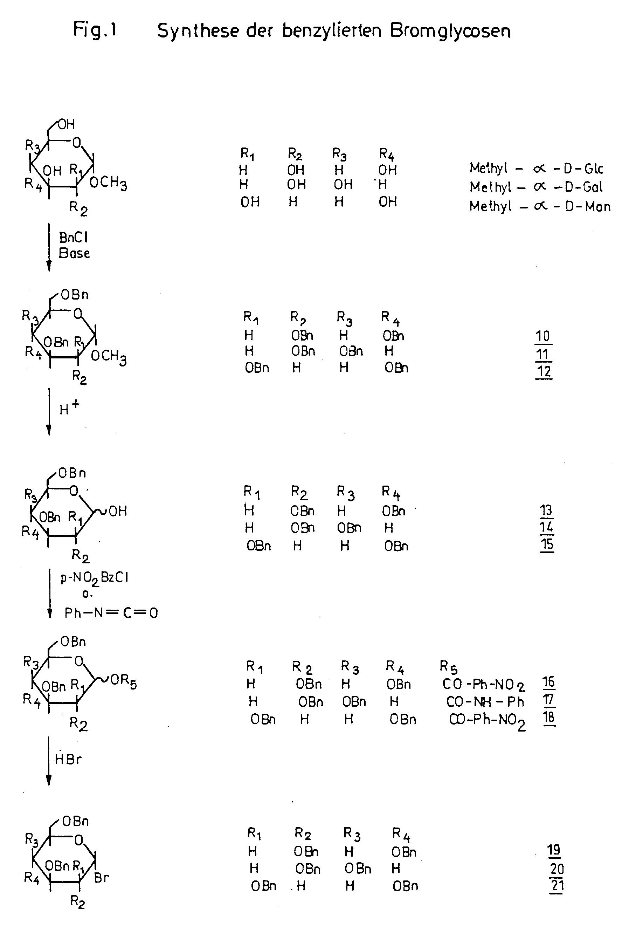

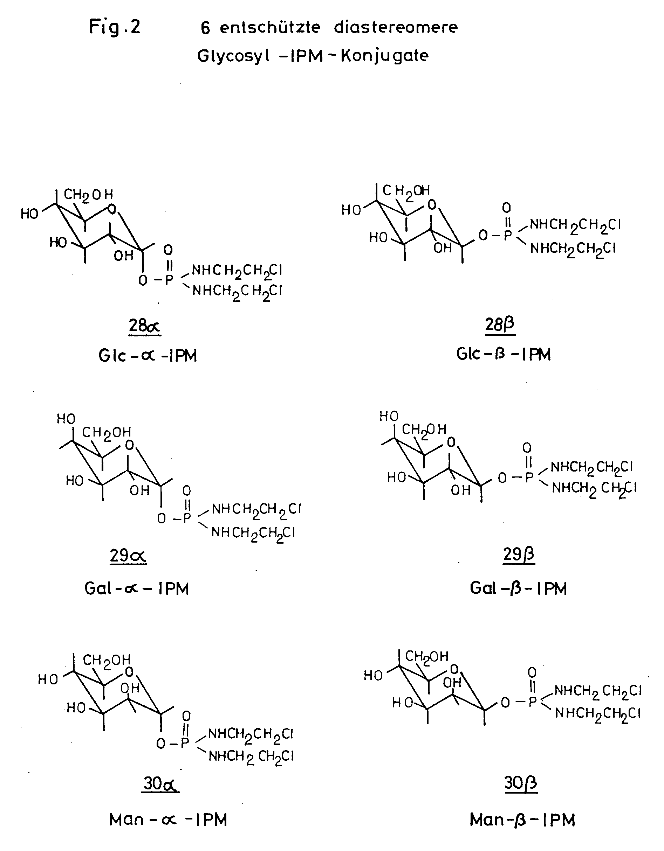

Diese beiden Verbindungen können jeweils mit geschützten Bromsacchariden nach der am Beispiel der Verbindung 28 β, Glucose-β IPM bwz. der Disaccharidverbindung 50 und 51 gemäß den beiliegenden Figuren 1 und 2 dargestellten Arbeitsweise erhalten werden.

Ein geschützter Zucker A wird mit einer Phosphorsäureverbindung B zur Reaktion gebracht, indem man beide in einem Lösungsmittel, vorzugsweise einem polaren Lösungsmittel, z.B. Acetonitril, CH₂Cl₂, oder Toluol im Temperaturbereich von 20°C bis 120°C über eine Zeitspanne von 1 h bis 48 h unter Zusatz einer Hilfsbase, z.B. Et₃N, Et(i-Prop)₂N aufeinander einwirken läßt. Die allgemeine Verfahrensvorschrift kann durch folgendes Formelschema dargestellt werden. 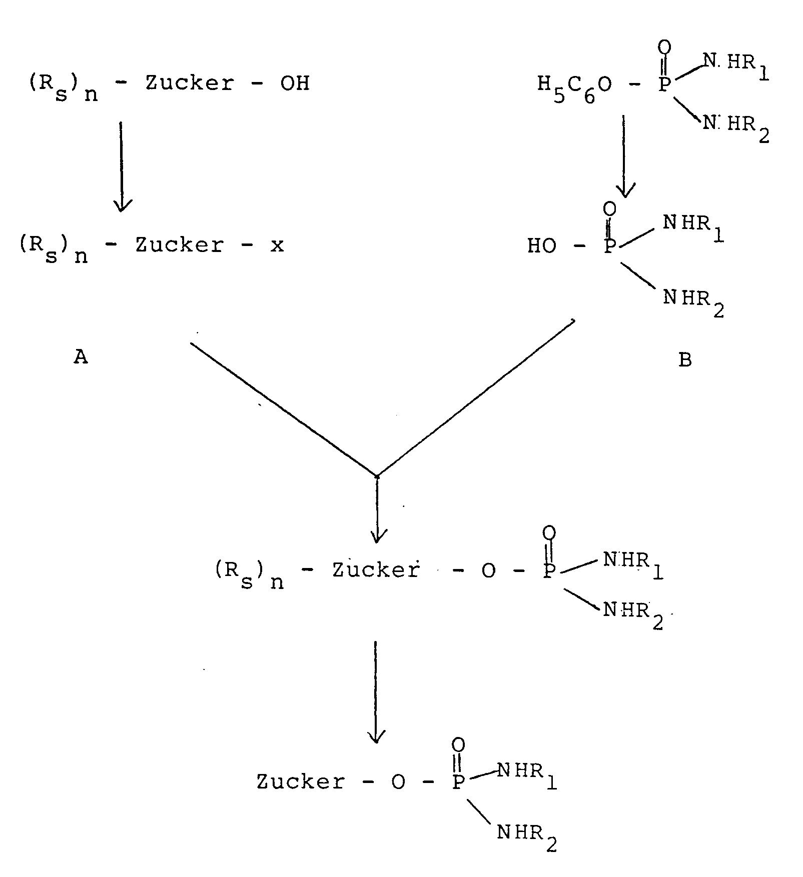
Die Reaktion mit Ifosfamid erfolgte in entsprechender Weise
Rₛ = eine Schutzgruppe, z.B. Benzyl,
x = eine reaktive Gruppe, z.B. Brom
Ausgehend von den 1-O-Methylpyranosiden der Glucose, Galactose und Mannose können die entsprechenden 2,3,4,6-Tetra-O-benyl-alpha-D-glycopyranosylbromide gemäß Fig. 1 hergestellt werden.

Die Benzylierung erfolgt in an sich bekannter Weise, z.B. in Dioxan mit Benzylchlorid in Gegenwart von KOH für Methyl-alpha-D-gluco- bzw. galactopyranosid, während Methyl-alpha-D-mannopyranosid, z.B. in Benzylchlorid/NaH umgesetzt wird. Die benzylierten Methylglycoside 10, 11 und 12 werden anschließend, z.B. mit H₂SO₄/HOAc zu 13 bzw. mit HCl/HOAc zu 14 und 15 hydrolysiert. Die Verbindung 13 und die isomere Mannose 15 können dann z.B. mit p-Nitrobenzoylchlorid in Dichlormethan/Pyridin zu den entsprechenden 2,3,4,6-Tetra-O-benzyl-1-O-p-nitrobenzoyl-D-glycopyranosen 16 und 18 derivatisiert werden, die beide durch Umkristallisieren bequem zu reinigen sind. Da dies für das p-Nitrobenzoat der 2,3,4,6-Tetra-O-benzylgalactose nicht so gut möglich ist, wird hier die Derivatisierung mit Phenylisocyanat in Pyridin zu kristallisierbarer 2,3,4,6-Tetra-O-benzyl-1-O-(N-phenyl-carbamoyl)-D-galactopyranose 17 vorgezogen (Kronzer und Schuerch, 1974, Carboh. Res. 33, 273).

Die benzylgeschützten alpha-Bromhalogenosen 19, 20 und 21 werden, z.B. durch Behandeln mit HBr in CH₂Cl₂ gebildet und nach üblicher Aufarbeitung, nämlich Abfiltrieren von p-Nitrosobenzoesäure bzw. Aniliniumbromid und Entfernen von überschüssigem HBr zweckmäßig direkt ohne weitere Reinigung in den nachfolgenden Glycosylierungsreaktionen eingesetzt.

Die benzylgeschützten Glycosyldonoren besitzen nun keinen Substituenten am C-2, der einen dirigierenden Einfluß auf die Glycosylierung ausüben könnte. Die Umsetzung der mit Benzylschutzgruppen versehenen bromierten Zucker mit Phosphoramid bzw. Ifosfamid erfolgte in Dichlormethan/Triethylamin. Die Ausbeute der Umsetzung von 16, 17 und 18 zu 22, 23 und 24 ist in Tabelle 1 angegeben, ebenso wie das Anomerenverhältnis und das Elutionsmittel bei der SC. Die Trennung gelingt z.B. mittels Säulenchromatographie.

**Tabelle 1**

| | Ausbeute (%) | α:β | EE:PE |
|---|---|---|---|
| 22 | 68 | 56:44 | 60:40 |
| 23 | 65 | 50:50 | 60:40 |
| 24 | 47 | 55:45 | 80:20 |

Bei dar HPLC-Analyse ist die sebr starke Konzentrationsabhängigkeit der Retentionszeiten, ebenso wie das starke tailing dar Konjugate zu beachten. Die Sicherung der Struktur und dar Stereocnemie erfolgte mittels ¹H- und ¹³C-NMR-Sepktroskopie.

Zur Herstellung größerer Mengen wurde eine Arbeitsweise verwendet, bei der durch stereoselektive Synthese ein Anomeres bevorzugt entsteht und man somit auf eine HPLC-Reinigung verzichten kann.

Gemäß Schmidt 1986, Angew. Chem. 98, 213, kann man benzylgeschützte O-Glycosyltrichloracetimidate als Glycosyldonoren für stereoselektive Snythesen einsetzen. Die Synthese der Trichloracetimidate erfolgte nach den in der Literatur beschriebenen Verfahren, ausgehend von den Tetra-O-benzylglycosen 13, 14 und 15 .

Unter Verwendung von Kaliumcarbonat als Base erhält man in einer kinetisch kontrollierten Reaktion aus 13 das β-Imidat 25β, mit NaH erzielt man eine schnelle Anomerisierung zum thermodynamisch stabileren 25alpha. Analog erhält man aus 14 das Galactosyl-imidat 26alpha, aus 15 das Mannosyl-imidat 27alpha. Diese Imidate bieten gegenüber den Brom-aktivierten Benzylglycosen den Vorteil der größeren Stabilität; sie sind gut isolierbar und lagerfähig.

So wurden die Imidate unter verschiedenen Reaktionsbedingungen mit IPM 4b umgesetzt. Als Lösungsmittel wurden z.B Dichlormethan oder Acetonitril verwendet, als Katalysatoren z.B. BF₃.Diethylether oder HCl in Dichlormethan.
Die Reaktion erfolgte schneller in CH₃CN als in CH₂Cl₂. Das Mannosyl-imidat 27alpha reagierte mit 4b selektiv zum Mannosid 24alpha.

Die geschützten Glycoside können z.B. durch katalytische Hydrierung mit Pd/Aktivkohle bei Raumtemperatur entschützt werden. Der Verlauf der Hydrierung läßt sich mittels DC verfolgen. Die Detektion kann mit methanolischer Schwefelsäure erfolgen, empfindlicher jedoch ist der Nachweis mit 4-(p-Nitrobenzyl)-pyridin-Reagenz (NBP).

Nach Beendigung der Reaktion wurde der Katalysator abfiltriert, das Filtrat einrotiert und im Hochvakuum getrocknet. Bei Einsatz der anomerenreinen Glycoside 22, 23 und 24 wurden die entsprechenden Glycoside 28, 29 und 30 ebenfalls anomerenrein erhalten (Fig.2 ).

Die entsprechenden Phosphoramid-konjugate sowie die Konjugate der Derivate der Formeln 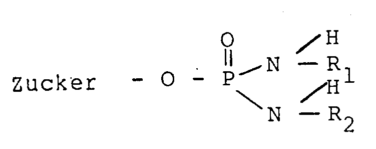
können in entsprechender Weise erhalten werden.

Eine Reinigung der als klare, farblose, hochviskose Öle anfallenden Produkte ist nicht notwendig. Sollten dennoch Nebenprodukte bei der Hydrierung entstehen, so können diese durch kurze SC an Kieselgel (Acetonitril/Methanol-Gemische) abgetrennt werden.

Die HPLC-Analyse erlaubt die Ermittlung der Anomerenverhältnisse im Falle des Glucosids 28 und des Mannosids 30.

**Tabelle 2**

| Reaktionen der Trichloracetimidate mit IPM | | | | | |
|---|---|---|---|---|---|
| Edukt | LM | Reaktionsbed. | Produkt | Ausbeute | α:β |
| 25α | CH₂Cl₂ | RT,1d | 22 | - | |
| | | RF,6h | | 56% | 1:6 |
| | CH₃CN | RT,1d | | gering | |
| | | RF,6h | | 42% | 1:20 |
| 25β | CH₂Cl₂ | RF, 4h | | 51% | 2:3 |
| | CH₃CN | RF, 6h | | 45% | 1:1.1 |
| 26α | CH₂Cl₂ | RT,4h | 23 | - | |
| | | RF,8h | | 48% | 2:5 |
| | CH₃CN | RF,6h | | 42% | 1:3 |
| 27α | CH₂Cl₂ | RT,4h | 24 | - | |
| | | RF,4h | | 48% | |
| | CH₃CN | RF,6h | | 47% | nur α |
| RT = Raumtemperatur | | | | | |

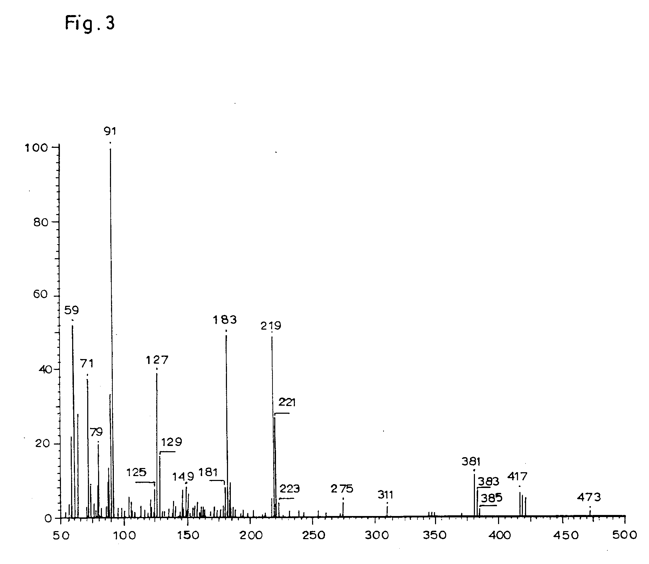

Die Identität der Verbindungen konnte durch FAB-MS und ¹H-NMR-Spektroskopie gesichert werden. Die Konfiguration der Zuckerreste wurde darüberhinaus durch enzymatische Reaktionen bestätigt. Als Beispiel ist in Fig.3 , das FAB-Sepektrum (negativ) von Glc-β-IPM 28β angegeben.

Unter Verwendung von Glycerin als Matrix lieferten sowohl negative als auch positive FAB-Spektren wesentliche Informationen. Von allen 6 Verbindungen waren Signale der negativen Molekülpeak-Ionen (M-H)⁻ bzw. der positiven Molekülpeak-Ionen (M+H)⁺ zu sehen. Es traten jeweils 3 Peaks in einem charakteristischen Verhältnis auf, ein Phänomen, das dadurch zustande kommt, daß das Molekül 2 Chloratome enthält, Chlor in der Natur jedoch nicht isotopenrein sondern im Verhältnis ³⁵Cl : ³⁷Cl - 3:1 vorkommt, was eine Relation der Isotopenpeaks von etwa 9:6:1 bedingt. So werden für die Ionen (M+H)⁺ die erwarteten Werte m/e = 383, 385, 387, für (M-H)⁻ entsprechend m/e = 381, 383 und 385 gefunden. Ein charakteristisches Fragmention, nämlich das des alkylierenden Aglycons IPM, wurde mit den Signalen m/e = 221, 223, 225 für (IPM+H)⁺, sowie für m/e = 219, 221 und 223 für (IPM-H)⁻ eindeutig nachgewiesen; auch hier findet sich die typische Verteilung der Isotopenpeaks. In Fig. 3 sind die beiden Tripletts der Ionen (M-H)⁻ und (IPM-H)⁻ von Glc-β-IPM 28β deutlichzu erkennen. Die Signale m/e = 91 und 183 stammen von der verwendeten Glycerinmatrix.

Die Zuordnung der Konfiguration am anomeren Zentrum gelang mittels ¹H-NMR wie bei den geschützten Edukten über die typischen Kopplungen des Protons H-1 und dessen chemischer Verschiebung; diese Parameter faßt Tab. 3 zusammen.

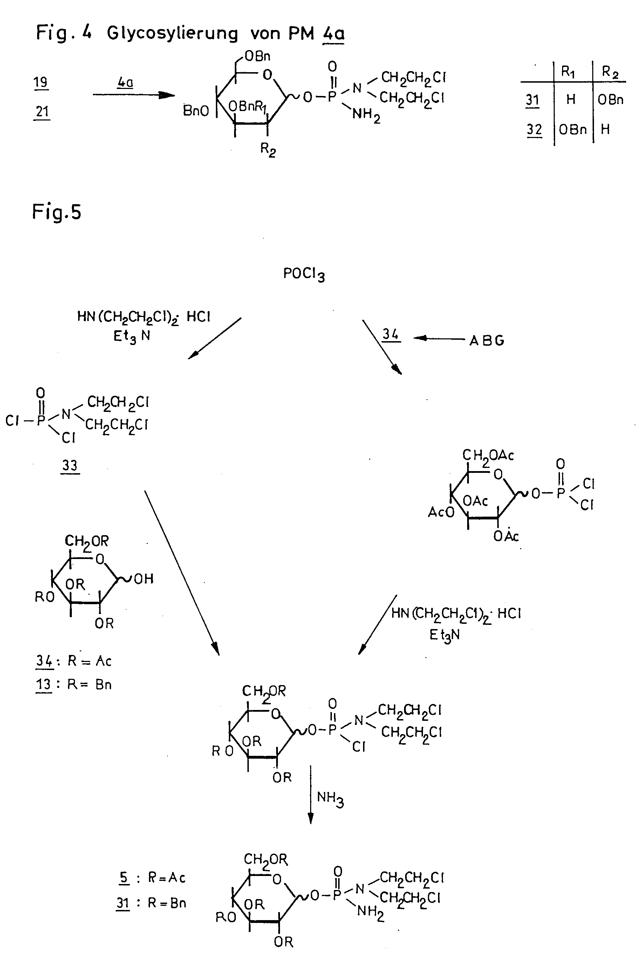

Der zu IPM isomere PM 4a reagierte mit 2,3,4,6-Tetra-O-benzyl-alpha-D-glucopyranosid 19 und dem entsprechenden Mannosyldonor 21 in Dichlormethan/Triethylamin unter Bildung von 2,3,4,6-Tetra-O-benzyl-D-glucopyrnosyl-N,N-bis-(2-chlorethyl)phosphorsäurediamid 31 bzw. zum 2-epimeren 32 Fig. 4 ).

**Tabelle 3**

| Chemische Verschiebungen und Kopplungen des anomeren Protons der entschützten Monosaccharid-IPM-Konjugate | | | |
|---|---|---|---|
| | δ(H-1) | J_{1,2} | J_{1,P} |
| 28α | 5.605 | 3.4 | 7.8 |
| 28β | 5.004 | 8.0 | 8.0 |
| 29α | 5.642 | 3.1 | 7.8 |
| 29β | 4.950 | 8.0 | 8.0 |
| 30α | 5.564 | 2.0 | 8.0 |
| 30β | 5.282 | 1.1 | 8.6 |

Zur Herstellung der Glycosyl-PM-Konjugate 5 oder 31 sind noch weitere Synthesewege möglich, wie dies Fig. 5 zeigt.

In entsprechender Weise (Beispiel 1 und 2) wurden über die Hepta-O-benzylglycosen 43 und 44

| | R₁ | R₂ |
|---|---|---|
| 43 | OBn | H |
| 44 | H | OBn |

die Disaccharid-IPM-Konjugate 50 und 51 erhalten.

| | R₁ | R₂ |
|---|---|---|
| 50 | OH | H |
| 51 | H | OH |

Tabelle 4 zeigt die ¹H-NMR-Daten der entschützten Disaccharid-Konjugate.

| | δ(H-1) | J_{1,2} | J_{1,P} | δ(H-1') | J_{1',2'} |
|---|---|---|---|---|---|
| 50α | 5.623 | 3.6 | 7.8 | 4.478 | 8.0 |
| 50β | 5.049 | 8.0 | 8.0 | 4.473 | 8.0 |
| 51α | 5.622 | 3.6 | 7.8 | 4.537 | 7.9 |
| 51β | 5.044 | 8.0 | 8.0 | 4.532 | 8.0 |

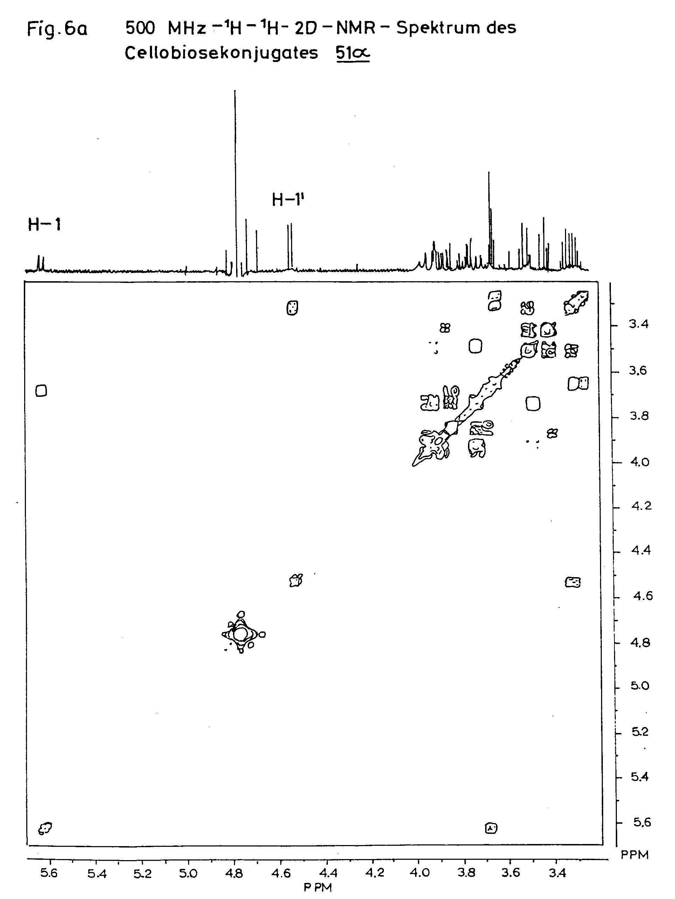

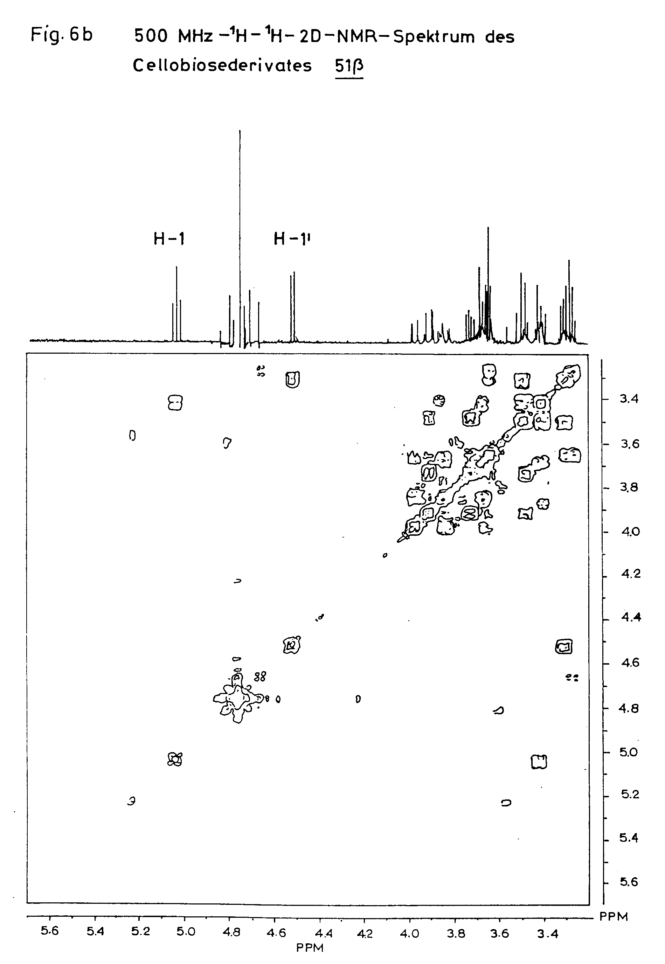

Auch hier wurde die Identität aller vier diastereomeren Verbindungen durch 2D-NMR-COSY gesichert. Fig. 6a/6b zeigen stellvertretend das 2D-Spektrum der entschützten Cellobiose-IPM-Konjugate 51-alpha und 51β.

Auch hier wurde die Struktur des Zuckerrestes durch enzymatische Reaktionen bestätigt, also Lactose bzw. Cellobiose.

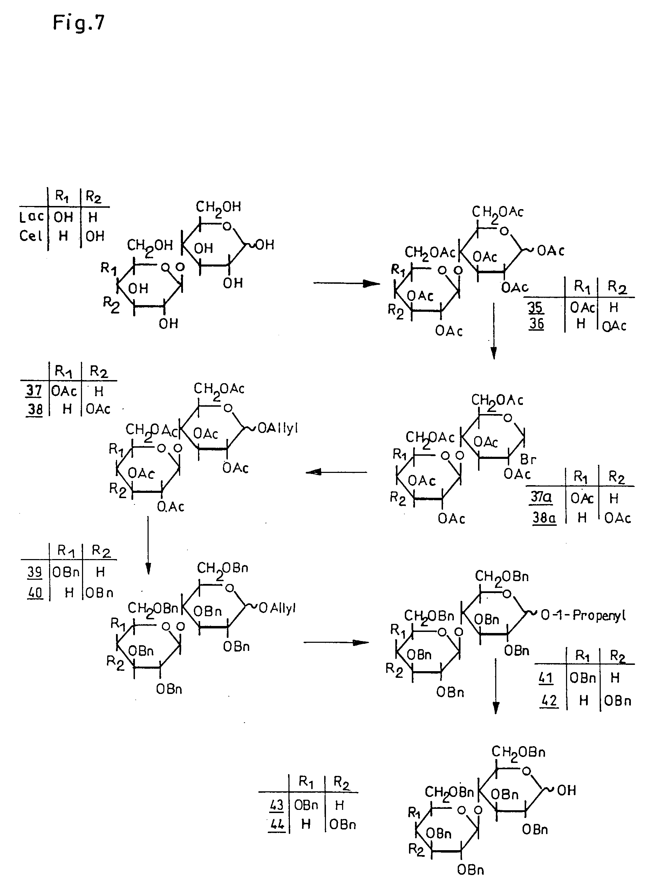

Beim Aufbau von Disaccharid-gekoppelten IPM-Konjugaten hat die Verwendung von natürlich vorkommenden Disacchariden wie Lactose oder Cellobiose als Edukt gegenüber einem Konjugataufbau aus Monozucker den Vorteil, daß die glycosidische Bindung zwischen den Zuckern vorgegeben ist und somit ein sterooselektriver Synthese schritt eingespart werden kann kann. Demzufolge wurden die vorher beschriebenen Syntheseverfahren auch auf die Herstellung Disaccharid-gekoppelter IPM-Konjugate angewandt. Es wurde also zuerst ein benzylgeschütztes Disaccharid benötigt, dessen 1-O-Position entweder durch Bromwasserstoff oder Trichloracetonitril aktiviert werden konnte. So wurden aus Lactose und Cellobiose in Anlehnung an das Verfahren von S. Koto (Koto et al., 1982, Nihon-Kagakkai: Nihon-Kagaku-Kaishi (J.Chem.Soc.Jpn.) 10,1651), die Disaccaridbausteine 2,3,6-2',3',4',6'-Hepta-O-benzyl-lactose und das isomere Cellobiosederivat 44 synthetisiert (Fig. 7).

Wurden die Glycoside in HEPES oder Wasser mit entsprechenden Glycosidasen inkubiert, so konnte die rasche Spaltung der glycosidischen Bindung beobachtet werden.

Die Konjugate Lac-IPM 50 und Cel-IPM 51 erwiesen sich in methanolischer Lösung, ebenso wie die Monosaccharidkonjugate 28-30, bei Raumtemperatur als stabil (DC-Analyse). Da in 50 und 51 je 2 glycosidische Bindungen vorliegen, die gleiche (51β) oder unterschiedliche Konfiguration (z.B. 50alpha) aufweisen, wurden zur Prüfung der enzymatischen Freisetzung von IPM neben einzelnen Glycosidasen auch Enzymgemische verwendet. Die enzymatische Spaltung der anomerenreinen Konjugate 50 und 51 wurde dünnschichtchromatographisch verfolgt; es wurden mit NBP entweder intakte Konjugate oder die Spaltprodukte 28alpha, 28β oder IPM detektiert.

Durch entsprechende Glycosidasen wurden alle Konjugate rasch gespalten und können z.B. den Metaboliten der allgemeinen Formel 1 bzw. 1a freisetzen.

Bei den Disaccharidkonjugaten 50 und 51alpha war zur IPM-Freigabe die Spaltung zweier verschiedener glycosydischer Bindungen nötig. Lediglich 51β, das zwei gleich konfigurierte Bindungen aufweist, konnte durch ein einziges Enzym, nämlich β-Glucosidase vollständig gespalten werden.

Dies wurde bestätigt durch die Messung der biologischen Wirksamkeit, nämlich der cytotoxischen Aktivität in vitro durch Untersuchung an einem Retothelsarcom der Maus und an Mammatumorzellinien der Ratte sowie an Eb/ESb-Zellinien.

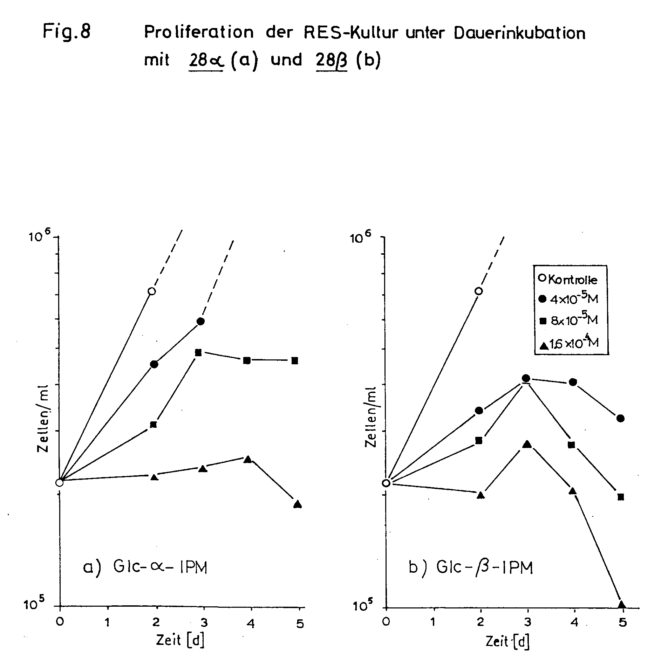

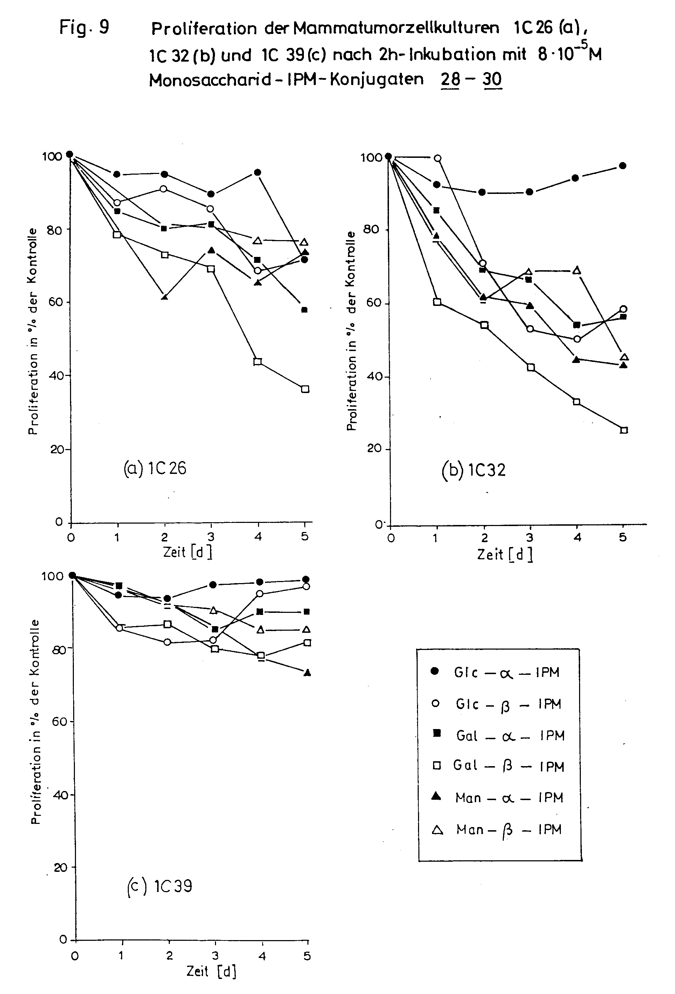

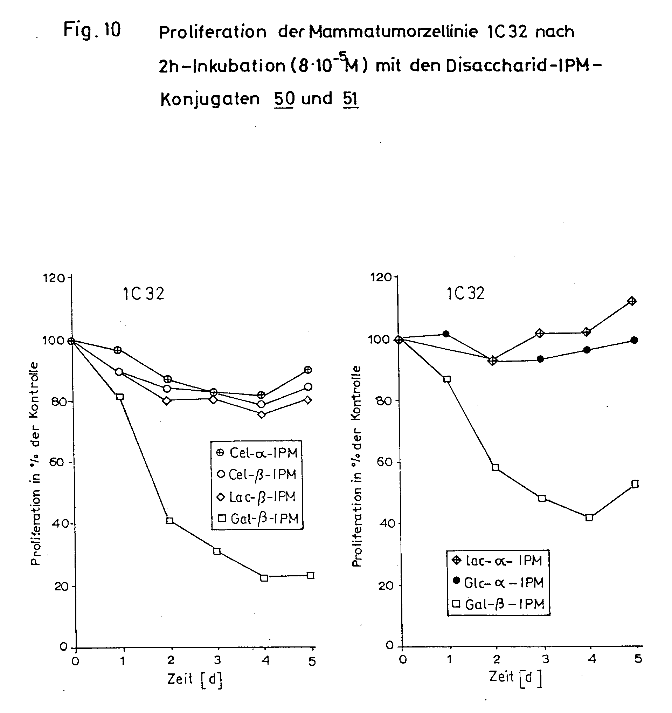

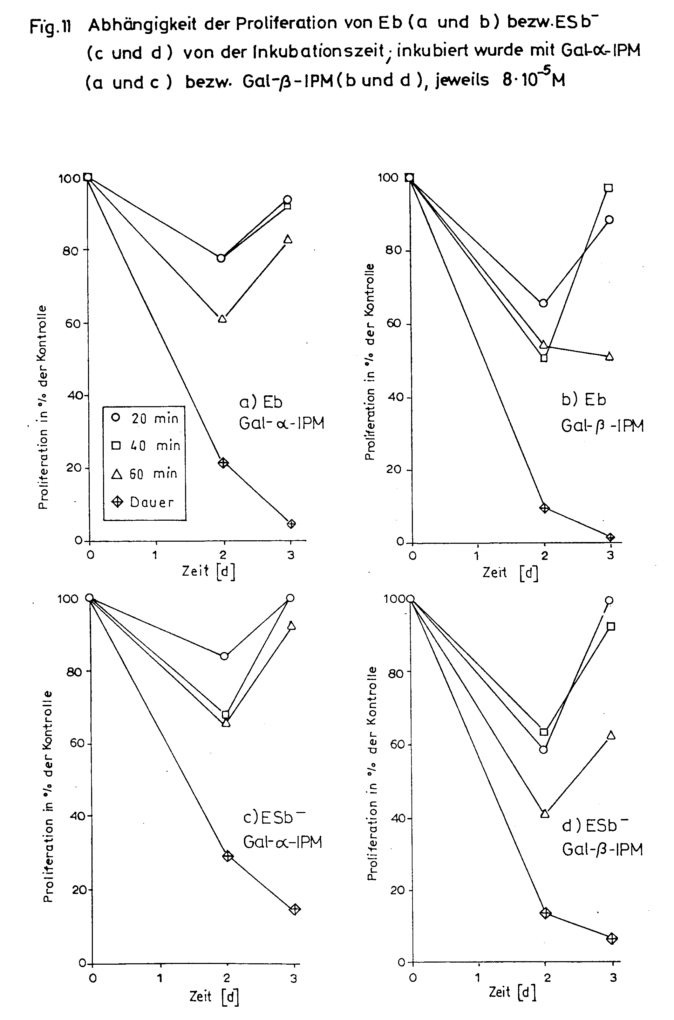

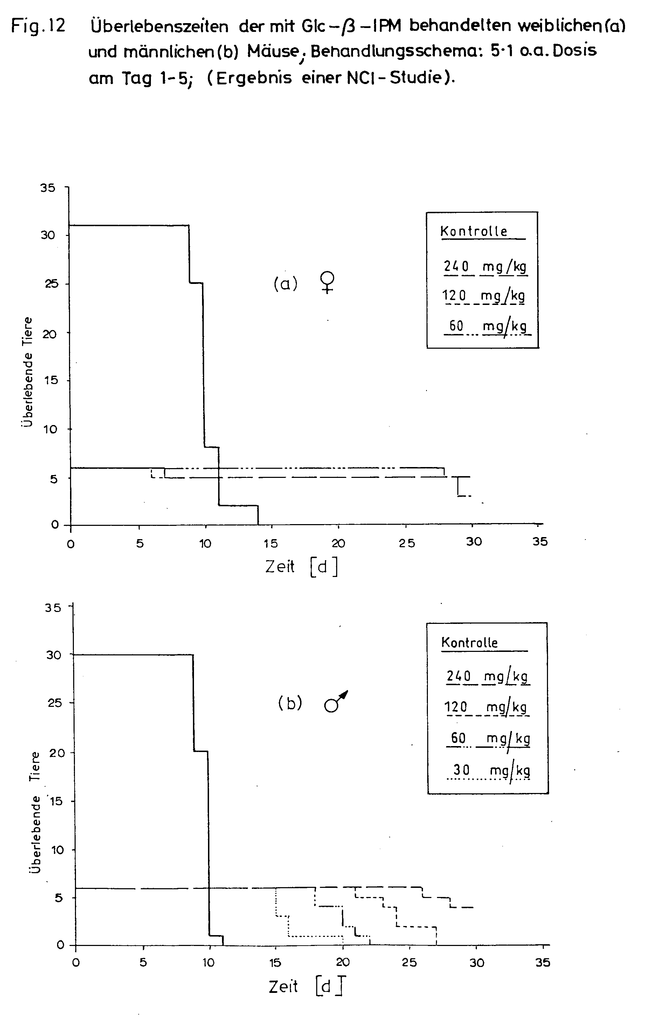

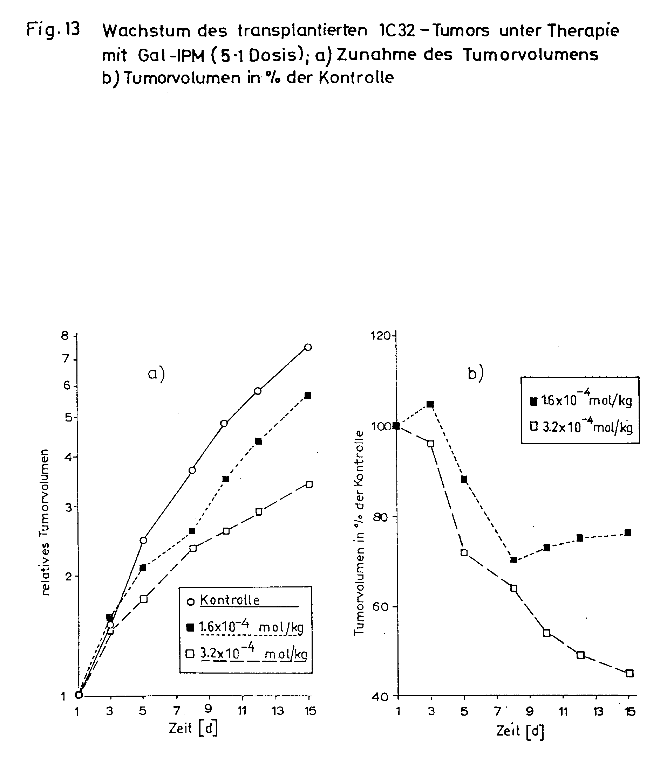

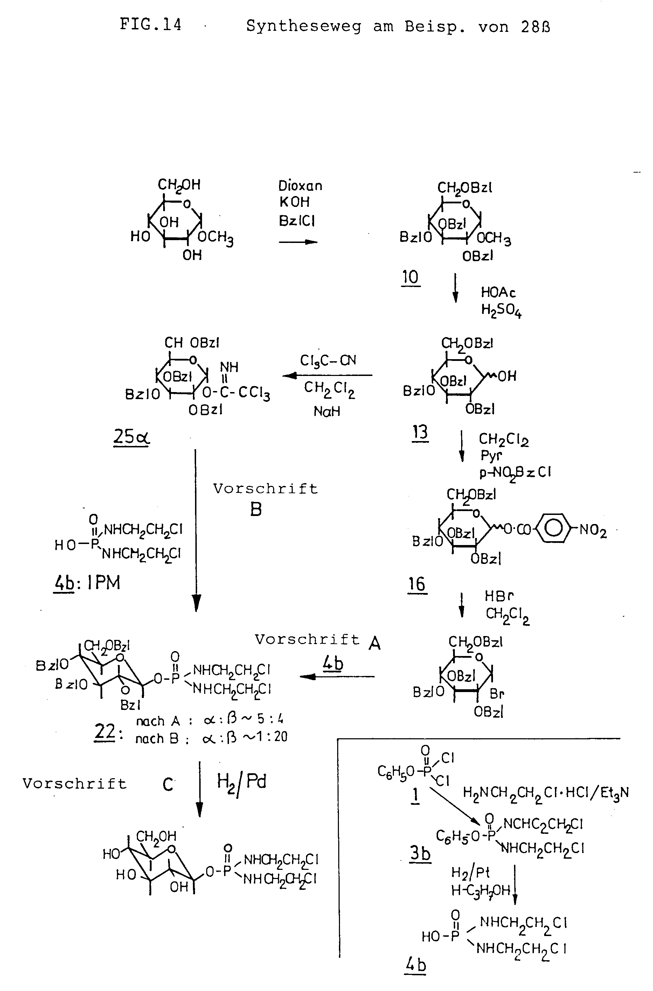

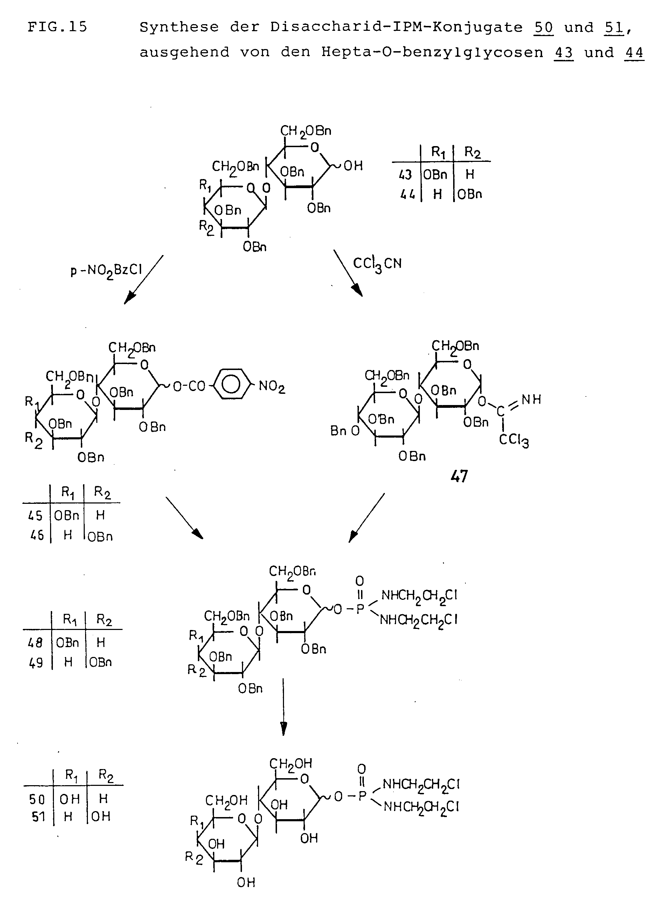

In vivo Untersuchungen erfolgten an der P388-Leukämie der Maus und am 1C32-Tumor der Ratte. Ergebnisse der in vitro und in vivo Untersuchungen sind in den Fig. 8 bis 13 ersichtlich.

Die Toxizitätmessung zeigte, daß bei der i.p.-Gabe von 100 mg/kg bzw. 1000 mg/kg Glc-β-IPM an männlichen CD₂F₁-Mäusen keine akute Toxizität zu beobachten war. Die Sektion der Tiere nach 28 Tagen blieb ohne Befund. Auch die BD6-Ratten mit transplantierten 1C32-Tumor, die mit Gal-IPM behandelt wurden (5-122,5 bzw. 5-245 mg/kg) ließen keine Toxizität erkennen. Das Sektionsergebnis (u.a. Leber, Nieren, Milz, Hirm, Knochenmark) am 15. Tag nach Beginn der Behandlung brachte keinen Befund.

Zusammenfassend ist also festzustellen, daß in vitro Untersuchungen an einem Retothelsarcom die Cytotoxität sowohl der Aglyca PM und IPM, als auch der Monosaccharid-IPM-Konjugate der allgemeinen Formel 1 bzw. 1a und auch der Disaccharide-Konjugate der allgemeinen Formel 1 bzw. 1a zeigen. Bei in vitro Tests treten bei den Mammatumorzellinien 1C29, 1C32 und 1C39 gewisse Abstufungen zu Tage, weil Gal-β-IPM immer die wirksamste Substanz ist. Auch in vivo Versuche zeigten eine gute Wirksamkeit am P388-Modell und bei dem soliden 1C32-Mammatumor, und zwar ohne akute Toxizität. Nachdem auch die Knochenmarkstoxizität, die am Beispiel Glc-β-IPM untersucht wurden, nur sehr gering ist, ist eine wichtige Forderung bei der Entwicklung neuer antineoplastisch wirksamer Chemotherapeutika erfüllt.

Die folgenden Beispiele erläutern im einzelnen die Herstellung verschiedener beispielhafter Verbindungen.

### Beispiel 1

### Allgemeine Arbeitsvorschift zur Herstellung der benzylgeschützten Glycosyl-Phosphorsäurediamide

### Vorschrift A: Aktivierung durch Bromwasserstoff

4,35 mmol der in an sich bekannter Weise geschützten p-Nitrobenzylglycose (z.B. 3,0 g 16) bzw. des N-phenylcarbamoylderivates 17 wurden nach Trocknung über P₂O₅ in 10 ml CH₂Cl₂ abs. gelöst (Dreihalskolben). Bei -20°C wurden unter trockenem Stickstoff 30 ml einer HBr-gesättigten CH₂Cl₂-Lsg. langsam eingespritzt. Nach wenigen Sekunden fällt p-Nitrobenzoesäure aus. Nach Erwärmen auf RT (Raumtemperatur) (innerhalb 30 min) wurde noch 1 h bei RT gerührt und anschließend durch eine Umkehrfritte in einen zweiten Dreihalskolben abgesaugt. Nach Abdampfen des CH₂Cl₂ (Rühren mit Magnetfisch, Wasserstrahlvakuum, Wasserbad, RT) wurden noch zweimal 5 ml Diethylether zugegeben und jeweils wie vorher beschrieben eingedampft um HBr-Reste zu entfernen. Das erhaltene gelb bis orange gefärbte Öl wurde nun in 20 ml CH₂Cl₂ gelöst und 1,0 g 4a oder 4b oder 54 (4,5 mmol) und 0,85 ml Triethylamin (6 mmol) zugegeben. Nach 3 d Rühren bei RT wurde filtriert, 2 x mit wenig Wasser gewaschen, die organ. Phase über Na₂SO₄ getrocknet und einrotiert. Anschließend erfolgte SC der meist gelben hochviskosen Öle und man erhielt schon Anreicherungen des jweiligen Anomeren in frühen bzw. späten Fraktionen. Vollkommene Anomerenreinheit konnte in allen Fällen durch präp. HPLC erzielt werden, manchmal auch durch Kristallisation.

Alle Arbeitsschritte wurden unter trockenem Stickstoff, mit trockenen Lösungsmitteln und unter Lichtausschluß durchgeführt.

### Vorschrift B: Umsetzung der Phosphorsäurediamide mit Glycosylimidaten

1.0 mmol Glycosylimidat (z.B. 25) wurde in 20 ml Acetonitril gelöst. Nach Zugabe von 1,0 mmol Phosphorsäurediamid wurde 6 h unter Rückfluß erhitzt (Lichtausschluß). Nach Filtration und Einrotieren wurde an Kieselgel in an sich bekannter Weise chromatographiert.

### Vorschrift C: Abspaltung der Benzylschutzgruppen

0.1 mmol der benzylgeschützten Monosaccharidderivate 22, 23, 24 oder 55 bzw. der Disaccharidkonjugate 48 oder 49 wurden in 15 ml MeOH gelöst. Nach Zugabe von ca. 5 mg Pd/Aktivkohle (oxidierte Form, Gehalt 10 % Pd) pro 0,1 mval abzuspaltender Benzylgruppe (also z.B. 20 mg Katalysator pro 0,1 mmol 22, 35 mg pro 0,1 mmol 48) wurde bei RT hydriert bis DC-analytisch die Abspaltung aller Schutzgruppen nachweisbar war (nach 1 - 4 h). Bei sorfortigem Abbruch der Hydrierung wurde das entschützte Produkt nach Filtration und Einroterien der Lösung sauber erhalten. Bei unnötig langer Reaktionszeit zersetzte sich das entschützte Glycosid und es bildete sich IPM 4b. In diesem Fall konnte das gewünschte Produkte nach SC (Kieselgel, CH₃CN:MeOH = 70:30) isoliert werden.
- DC:: Kieselgel, CH₃CN:MeOH = 30:70, Rf(28α) = 0.58, Rf(4b) = 0.18
Kieselgel, CH₃CN:MeOH:H₂O = 75:20:5
- Rf-Werte:: 0.44 (28α), 0.48 (28β)
0.43 (29α), 0.45 (29β)
0.43 (30α), 0.41 (30β)
0.29 (50 und 51), 0.13 (4b).

Zur Detektion wurde mit NBP-Reagenz (2.5%ig in Aceton) besprüht, 10 min auf 120 °C erhitzt und nach dem Abkühlen auf RT mit 0.5 M-NaOH besprüht. Die IPM-Konjugate färbten sich tiefblau, während IPM selbst hellblau erschien.

Phosphoramide mustard und Ifosfamide mustard wurden nach aus der Literatur bekannten Arbeitsweisen hergestellt.

### Monosaccharid-PM/IPM-Konjugate (analog 6)

Ansatz: 0,55 g 4a (2,5 mmol) (N,N-Di-(2-chorethyl)phosphorsäurediamid)
1,03 g ABG (2,5 mmol)
Ausbeute: 30 mg 5 als gelbliches, klares Öl (2.2% d. Th.), Diastereomerengemisch (A+B, s. NMR-Spektrum)

| | | C | H | N |
|---|---|---|---|---|
| Analyse C₁₈H₂₉Cl₂N₂O₁₁P (551.3) | ber. | 39.24 | 5.30 | 5.08 |
| | gef. | 39.53 | 5.38 | 4.94 |

mit D₂O, -NH₂), 3.35-3.70 (m, 8H, 2x -CH₂CH₂Cl), 3.82 (m, 1H, H-5) 4.148 (dd, H-6a(A), J_{5,6a}=5.0 Hz, J_{6a,6b}=12.5 Hz), 4.208 (dd, H-6a(B), J_{5,6a}=4.9 Hz, J_{6a,6b}=12.5 Hz), 4.244 (dd, H-6b(A), J_{5,6b}=2.1 Hz, J_{6a,6b}=12.5 Hz), 4.288 (dd, H-6b(B), J_{5,6b}=2.1 Hz, J_{6a,6b}=12.5 Hz). 5.02-5.12 (m, 2H), 5.21 und 5.23 (2t, zusammen 1H, (B+A), J=9.5 Hz), 5.298 und 5.316 (2t, zusammen 1H, H-1(A) und H-1(B), J_{1,2}=J_{1,P}=7.8 Hz).
MS: m/e = 331 (M-PM)

### 2,3,4,6-Tetra-O-acetyl-β-D-glucopyranosyl-N,N'-Di-(2-chlorethyl)-phosphorsäurediamid 6

Zu einer Mischung von 1.1 g 4b (5 mmol) und 2.05 g Acetobromglucose (5 mmol) in 50 ml trockenem Aceton wurden 0.5 g Triethylamin (5 mmol) zugetropft. Es wurde 48 h unter Lichtausschluß bei RT gerührt. Nach Filtration und Einrotieren wurde der Rückstand in CH₂Cl₂ aufgenommen, mit wenig 0.1 M HCl, ges. NaHCO₃-Lsg. und Wasser gewaschen, über Na₂SO₄ getrocknet und über Kieselgel chromatographiert (Aceton:n-Hexan = 40:60). Es wurden 80 mg 6 (2.9% d. Th.) als klares, farbloses Öl erhalten, das nach Monaten bei +4 °C kristallisierte.
Fp.: 91 °C
DC: Kieselgel, Aceton:n-Hexan = 1:1, Rf = 0.13

| | | C | H | N | Cl |
|---|---|---|---|---|---|
| Analyse C₁₈H₂₉Cl₂N₂O₁₁P (551.3) | ber. | 39.24 | 5.30 | 5.08 | 12.87 |
| | gef. | 39.42 | 5.40 | 4.80 | 12.58 |

### Penta-O-pivaloyl-β-D-glucose 7 (nach Kunz und Harreus, 1982) Liebigs Ann. Chem., 41

Ansatz: 29 g D-Glucose (0.16 mol) 121.2 g Pivaloylchlorid (1.0 mol) 200 ml Chloroform, 120 ml Pyridin
Ausbeute: 66 g 7 (69% d. Th.)
Fp.: 154 °C (Lit.: 156-158 °C)

| | | C | H |
|---|---|---|---|
| Analyse C₃₁H₅₂O₁₁ (600.8) | ber. | 61.98 | 8.72 |
| | gef. | 62.16 | 8.79 |

### 2,3,4,6-Tetra-O-pivaloyl-α-D-glucopyranosylbromid 8 (nach Kunz und Harreus, 1982) Liebigs Ann. Chem., 41

Ansatz: 12 g 7 (20mmol)
20 ml HBr in Eisessig (33%ig)
20 ml Dichlormethan
Ausbeute: 6.4 g 8 (55.5% d.Th.)
Fp.: 142 °C

| | | C | H |
|---|---|---|---|
| Analyse C₂₆H₄₃BrO₉ (579.5) | ber. | 53.89 | 7.48 |
| | gef. | 54.24 | 7.94 |

J_{2,3}=9.5 Hz), 5.21 und 5.64 (2t, 2H, J=9.5 Hz, H-4 und H-3), 6.62 (d, 1H, H-1, J_{1,2}=4.2 Hz).

### 2,3,4,6-Tetra-O-pivaloyl-β-D-glucopyranosyl-N,N'-Di-(2-chlorethyl)-phosphorsäurediamid 9

Zu einer Mischung von 0.38 g 4b (1.725 mmol) und 1.0 g 8 (1.725 mmol) in 50 ml Aceton wurden bei RT 0.5 g Ag₂CO₃ (1.8 mmol) gegeben. Nach 24 h Rühren (Lichtausschluß) bei RT wurde filtriert, eingeengt und über Kieselgel chromatographiert (Aceton:n-Hexan = 25:75). Es wurden 160 mg 9 (12.9% d. Th. ) als klares, farbloses Öl erhalten, das nach Monaten bei +4 °C kristallisierte.
Fp.: 94 °C
DC: Kieselgel, Aceton:n-Hexan = 1:1, Rf = 0.43

| | | C | H | N | Cl |
|---|---|---|---|---|---|
| Analyse C₃₀H₅₄Cl₂N₂O₂P (719.54) | ber. | 50.08 | 7.42 | 3.89 | 9.86 |
| | gef. | 51.35 | 7.98 | 3.24 | 9.36 |

### Methyl-2,3,4,6-tetra-O-benzyl-α-D-glucopyranosid 10 (nach Methods in Carbohydrate Chemistry, 1972)

Ansatz: 50 g Methyl-α-D-Glucopyranosid (257 mmol)
250 g KOH (gepulvert)
150 ml Dioxan
318 ml Benzylchlorid (2.76 mol)
Ausbeute: 116 g 10 (81.3% d. Th.) als hochviskoses, gelbes, klares Öl

| | | C | H |
|---|---|---|---|
| Analyse C₃₅H₃₈O₆ (554.68) | ber. | 75.79 | 6.91 |
| | gef. | 76.19 | 6.93 |

### Methyl-2,3,4,6-tetra-O-benzyl-α-D-galactopyranosid 11 (analog 10)

Ansatz: 40 g Methyl-α-D-galactopyranosid (206 mmol)
200 g KOH, gepulvert
200 ml Dioxan
280 ml Benzylchlorid
Ausbeute: 102 g 11 (89.3% d. Th.) als gelbes, klares Öl

| | | C | H |
|---|---|---|---|
| Analyse C₃₅H₃₈O₆ (554.68) | ber. | 75.79 | 6.91 |
| | gef. | 76.07 | 6.67 |

### Methyl-2,3,4,6-tetra-O-benzyl-α-D-mannopyranosid 12 (nach Koto et al., 1976)

- Ansatz:: 18 g Methyl-α-D-mannopyranosid (92.7 mmol)
81 g NaH-Suspension (20%ig)
450 ml Benzylchlorid

Rohausbeute: Nach dem Waschprozeß bilden sich 2 Phasen. Nach Abtrennen der oberen farblosen Phase (Weißöl) wurden 52 g gelbes, leicht getrübtes Öl erhalten, das ohne weitere Reinigung zu 15 umgesetzt wurde. Ein Teil des Öles wurde chromatographiert (Kieselgel, EE:PE = 40:60): gelbes, klares Öl.
- ¹H-NMR:: 90 MHz, CDCl₃
δ = 3.31 (s, 3H, -OMe), 3.65-4.15 (m, 6H, Zuckerprotonen), 4.43-5.10 (m, 9H, H-1 und 4×-CH₂-Ph), 7.1-7.45 (m, 20H, 4×-Ph).

### 2,3,4,6-Tetra-O-benzyl-α-D-glucopyranose 13 (nach Methods in Carbohydrate Chemistry, 1982)

Ansatz: 115 g 10 (207 mmol)
Ausbeute: 54 g 13 (48.3% d. Th.)
Fp.: 152 °C (aus Methanol umkristallisiert)

| | | C | H |
|---|---|---|---|
| Analyse C₃₄H₃₆O₆ (540.66) | ber. | 75.53 | 6.71 |
| | gef. | 75.69 | 6.91 |

### 2,3,4,6-Tetra-O-benzyl-α-D-galactopyranose 14 (nach Kronzer und Schuerch, 1974) Carboh. Res. 33, 273

- Ansatz:: 30 g 11 (54 mmol)
500 ml Essigsäure (80%ig)
150 ml 1 N-HCl
- Ausbeute:: 28.6 g 14 (98% d. Th.), gelbes, Klares Öl
- ¹H-NMR:: 90 MHz, CDCl₃
δ = 3.47 (d, 1H, austauschbar mit D₂O, -OH), 3.5-4.3 (m, 6H, Zuckerprotonen), 4.35-5.05 (m, 8H, 4×-CH₂-Ph), 5.28 (dd, 1H, H-1, J_{1,2}=3.2 Hz), 7.1-7.5 (m, 20H, 4×-Ph).

### 2,3,4,6-Tetra-O-benzyl-D-mannopyranose 15

50 g der Mischung 12 (ca. 90 mmol, enthält noch etwas Weißöl) wurden in 800 ml Eisessig gelöst und auf 80-85 °C erwärmt. Innerhalb 60 min wurden 120 ml 2 N-HCl zugetropft; nach weiteren 90 min wurden 200 ml Wasser zugesetzt, der Ansatz auf RT abgekühlt und anschließend mit 3 200 ml Toluol extrahiert. Die organ. Phase wurde mit ges. NaHCO₃-Lsg. und Wasser gewaschen, über Na₂SO₄ getrocknet und einrotiert. Der erhaltene braune Sirup wurde chromatographiert (Kieselgel, EE:PE = 30:70) und ergab 33.2 g 15 (68.3% d. Th.) als gelbes Öl.
- ¹H-NMR:: 90 MHz, CDCl₃
δ = 3.5-4.3 (m, 7H, 1H austauschbar mit D₂O, -OH und 6 Zuckerprotonen), 4.35-5.05 (m, 8H, 4×-CH₂-Ph), 5.22 (d, 1H, H-1, J_{1,2}∼2 Hz), 7.05-7.4 (m, 20H, 4×-Ph).

### 2,3,4,6-Tetra-O-benzyl-1-O-p-nitrobenzoyl-D-glucopyranose 16 (nach Methods in Carbohydrate Chemistry, 1972)

- Ansatz:: 15.5 g 13 (28.7 mmol)
6 g p-Nitrobenzoylchlorid (32.3 mmol)
3.75 ml Pyridin
- Ausbeute:: 15.6 g 16 (78.8% d. Th.) als weißes Pulver
- Fp.:: 93 - 98°C (aus Ethanol)

Erneutes Umkristallisieren aus Diisopropylether liefert 16α als Nadeln.
Fp.: 122 °C

| | | C | H | N |
|---|---|---|---|---|
| Analyse C₄₁H₃₉NO₉ (689.76) | ber. | 71.39 | 5.70 | 2.03 |
| | gef. | 71.37 | 5.67 | 2.04 |

Aus der Mutterlauge kristallisierte 16β, Fp.: 98 °C

### 2,3,4,6-Tetra-O-benzyl-1-O-(N-phenylcarbamoyl)-D-galactopyranose 17 (nach Kronzer und Schuerch, 1974) Carboh. Res. 33, 273

Ansatz: 18.7 g 14 (34.6 mmol)
50 ml Pyridin
5.4 ml Phenylisocyanat
Ausbeute: 8.0 g 17 (35% d. Th.), (α:β = 35:65)
Fp.: 120-122 °C (aus Ethanol)

| | | C | H | N |
|---|---|---|---|---|
| Analyse C₄₁H₄₁NO₇ (659.78) | ber. | 74.64 | 6.26 | 2.12 |
| | gef. | 74.21 | 5.97 | 2.36 |

Aus der Mutterlauge kristallisiert 17α, Fp.: 143 °C

### 2,3,4,6-Tetra-O-benzyl-1-O-p-nitrobenzoyl-α-D-mannopyranose 18 (nach Koto et al., 1976) Bull. Chem. Soc. Jpn. 49, 2639

Ansatz: 9.8 g 15 (18.1 mmol)
4.9 g p-Nitrobenzoylchlorid
50 ml Pyridin
Ausbeute: Nach Flash-Chromatographie (Kieselgel 60, EE:PE = 25:75) kristallisierten 7.7 g 18 (61.7% d. Th.) aus Diisopropylether.
Fp.: 105 °C

| | | C | H | N |
|---|---|---|---|---|
| Analyse C₄₁H₃₉NO₉ (689.76) | ber. | 71.39 | 5.70 | 2.03 |
| | gef. | 71.46 | 5.58 | 1.90 |

### 2,3,4,6-Tetra-O-benzyl-N,N'-Di-(2-chlorethyl)-phosphorsäurediamid 22

### 1.) nach Vorschrift A

Ansatz: 3.0 g 16 (4.35 mmol)
1.0 g 4b (4.6 mmol)
Ausbeute: nach SC (Kieselgel, EE:PE = 60:40) 2.2 g 22, (68% d. Th.), Anomerengemisch: α:β = 5:4 (¹H-NMR).
DC: Kieselgel, EE:PE = 60:40, Rf = 0.28, Rf(α) > Rf(β)
HPLC, anal.: Kieselgel, EE:Hexan = 75:25, Flow 1.0 ml/min, Rt(α) = 12.53', Rt(β) = 14.04'
HPLC, präp.: Kieselgel, EE:Hexan:MeOH = 58:42:0.5, Flow 10.0 ml/min, Rt(α) = 45', Rt(β) = 55'

| | | C | H | N | Cl | |
|---|---|---|---|---|---|---|
| Analyse C₃₈H₄₅Cl₂N₂O₇P (743.60) | ber. | 61.38 | 6.10 | 3.77 | 9.54 | |
| | gef. | 61.15 | 6.22 | 3.78 | 9.61 | 22α |
| | gef. | 60.82 | 6.29 | 3.61 | 9.37 | 22β |

### 2.) nach Vorschrift B

- Ansatz:: 4.5 g 25α (6.57 mmol)
1.45 g 4b (6.57 mmol)
- Ausbeute:: nach SC (s.o.): 2.06 g 22 (42.2% d. Th.), Anomerenverhältnis α:β ∼ 1:20 (nach ¹H-NMR und HPLC).
- Ansatz:: 50 mg 25β (0.073 mmol)
16.1 mg 4b (0.073 mmol)

DC zeigt Produkt mit Rf=0.28 und wenig Tetrabenzylglucose 13 sowie wenig Edukt 25β, Anomerenverhältnis α:β = 1:1.1 (HPLC)

### 2,3,4,6-Tetra-O-benzyl-D-galactopyranosyl-N,N'-Di-(2-chlorethyl)-phosphosäurediamid 23

### 1.) nach Vorschrift A

Ansatz: 2.6 g 17 (3.94 mmol)
0.9 g 4b (4.07 mmol)
Ausbeute: nach SC (Kieselgel, EE:PE = 60:40): 1.9 g 23 (65% d. Th.), Anomerengemisch α:β = 1:1 (¹H-NMR).
DC: Kieselgel, EE:PE = 60:40, Rf = 0.27, Rf(α)> Rf(β)
HPLC, anal.: Kieselgel, EE:Hexan = 75:25, Flow 1.0 ml/min: Rt(α) = 14.26', Rt(β) = 18.03'
HPLC, präp.: Kieselgel, EE:Hexan:MeOH = 58:42:0.5, Rt(α) = 51', Rt(β) = 62'

| | | C | H | N | Cl | |
|---|---|---|---|---|---|---|
| Analyse C₃₈H₄₅Cl₂N₂O₇P (743.60) | ber. | 61.38 | 6.10 | 3.77 | 9.54 | |
| | gef. | 61.26 | 6.19 | 3.76 | 9.76 | 23α |
| | gef. | 61.16 | 6.26 | 3.76 | 9.66 | 23β |

### 2.) nach Vorschrift B

- Ansatz:: 685 mg 26α (1.0 mmol)
221 mg 4b (1.0 mmol)
- Ausbeute:: Im filtrierten Reaktionsgemisch betrug das Anomerenverhältnis α:β = 55:45 (HPLC). Nach SC (s.o) wurden 260 mg 23 (38% d. Th.) erhalten.

### 2,3,4,6-Tetra-O-benzyl-D-mannopyranosyl-N,N'-Di-(2-chlorethyl)-phosphorsäurediamid 24

### 1.) nach Vorschrift A

Ansatz: 2.3 g 18 (3.33 mmol)
0.75 g 4b (3.39 mmol)
Ausbeute: nach SC (Kieselgel, EE:PE = 80:20)1.16 g 24 (47% d. Th.), Anomerengemisch: α:β = 55:45 (¹H-NMR)
DC: Kieselgel, EE:PE = 60:40, Rf(α) = 0.23, Rf(β) = 0.19
HPLC, anal.: Kieselgel, EE:Hexan = 75:25, Flow 1.0 ml/min, Rt(α) = 13.05', Rt(β) = 21.61'
HPLC, präp.: Kieselgel, EE:Hexan:MeOH = 64:36:0.5, Flow 10.0 ml/min, Rt(α) = 45', Rt(β) = 70'

| | | C | H | N | |
|---|---|---|---|---|---|
| Analyse C₃₈H₄₅Cl₂N₂O₇P (743.60) | ber. | 61.38 | 6.10 | 3.77 | |
| | gef. | 60.98 | 6.32 | 3.52 | 24α |
| | gef. | 60.98 | 6.19 | 3.29 | 24β |

### 2.) nach Vorschrift B

- Ansatz:: 3.2 g 27α (4.67 mmol)
1.04 g 4b (4.70 mmol)
- Ausbeute:: DC zeigt fast quantitative Umsetzung zu 24α mit Rf = 0.23. Im filtrierten Reaktionsgemisch findet sich nur das α-Anomere (HPLC). Nach SC wurden 1.6 g 24α (45% d. Th.) erhalten.

### O-(2,3,4,6-Tetra-O-benzyl-α-D-glucopyranosyl)-trichloracetimidat 25α (nach Schmidt und Stumpp, 1983) Liebigs Ann. Chem., 1249

- Ansatz:: 9.2 g 13 (17 mmol)
7.7 ml Trichloracetonitril
680 mg NaH
- Ausbeute:: 10.9 g 25α (93.6% d. Th.), farbloses klares Öl.
- DC:: Kieselgel, PE:E = 1:1, Rf = 0.44
- ¹H-NMR:: 90 MHz, CDCl₃.
δ = 3.6-5.1 (m, 14H), 6.51 (d, 1H, H-1, J_{1,2}=3.5 Hz), 6.95-7.55 (m, 20H, 4× -Ph), 8.55 (s, 1H, -NH-).

### O-(2,3,4,6-Tetra-O-benzyl-β-D-glucopyranosyl)-trichloracetimidat 25β (nach Schmidt et al., 1984) Liebigs Ann. Chem., 680

- Ansatz:: 1.3 g 13 (2.4 mmol)
1.25 g K₂CO₃ (getrocknet)
1.25 ml Trichloracetonitril
- Ausbeute:: nach Kieselgelfiltration: leicht gelbliches Öl (1.6 g, 97% d. Th., α:β = 1:5); nach SC (Kieselgel, E:PE = 2:3) wurde reines 25β als farbloses Öl erhalten (50 mg, 30% d. Th.).
- DC:: Kieselgel, PE:E = 1:1, Rf = 0.37
- ¹H-NMR:: 90 MHz, CDCl₃
δ = 3.55-3.90 (m, 6H), 4.40-5.05 (m, 8H), 5.82 (d, 1H, H-1), 7.1-7.5 (m, 20H, 4× -Ph), 8.70 (s, 1H, -NH-).

### O-(2,3,4,6-Tetra-O-benzyl-D-galactopyranosyl)-trichloracetimidat 26 (nach Schmidt et al., 1984) Liebigs Ann. Chem., 1343

- Ansatz:: 1.5 g 14 (2.77 mmol)
1.4 ml Trichloracetonitril
80 mg NaH
- Ausbeute:: nach Kieselgelfiltration wurde das Anomerenverhältnis zu α:β = 4:1 bestimmt (¹H-NMR: 90 MHz, CDCl₃, Nr. H14208, δ = 5.72 (d, 0.2H, H-1 (β), J_{1,2}=8.0 Hz), 6.52 (d, 0.8 H, H-1 (α), J_{1,2}=3.7 Hz), 8.51 (s, 0.8H, -NH- (α), austauschbar mit D₂O), 8.60 (s, 0.2H, -NH- (β), austauschbar mit D₂O).
Nach SC (Kieselgel, E:PE = 1:1) wurden 2 Fraktionen erhalten:
- F1:: 1130 mg 26α (59.5% d. Th.) als farbloses Öl, ¹H-NMR: 500 MHz, CDCl_{3,} Nr. H14112
- F2:: 320 mg 26 (16.8% d. Th.) als gelbliches Öl (α:β = 2:1) ¹H-NMR: 500 MHz, CDCl_{3,,} Nr. H14113
- DC:: Kieselgel, PE:E = 1:1, Rf(α) = 0.43, Rf(β) = 0.34

### O-(2,3,4,6-Tetra-O-benzyl-α-D-mannopyranosyl-trichloracetimidat 27α (nach Schmidt et al., 1984) Liebigs Ann. Chem., 1343

- Ansatz:: 4.5 g 15 (8.27 mmol)
4 ml Trichloracetonitril
45 mg NaH
- Ausbeute:: nach SC: 4.45 g 27α (65% d. Th.) als farbloses Öl
- DC:: Kieselgel, E:PE = 3:2, Rf = 0.51
- ¹H-NMR:: 90 MHz, CDCl₃
δ = 3.65-5.0 (m, 14H), 6.33 (d, 1H, H-1, J_{1,2}∼1.5 Hz), 7.0-7.5 (m, 20H, 4× -Ph), 8.52 (s, 1H, -NH-).

### α-D-Glucopyranosyl-N,N'-Di-(2-chlorethyl)-phosphorsäurediamid 28α

### Hydrierung von 22α nach Vorschrift C

- ¹H-NMR:: 500 MHz, D₂O, Nr. 13943
δ = 3.25-3.32 (m, 4H, 2× -CH₂-), 3.488 (t, 1H, J=9.5 Hz), 3.61-3.67 (m, 5H mit 2×-CH₂), 3.719 (t, 1H, J∼9.5 Hz), 3.75-3.90 (m, 3H), 5.605 (dd, 1H, H-1, J_{1,2}=3.4 Hz, J_{1,P}=7.8 Hz).
- FAB-MS:: Nr. MSN 13608
positiv: m/e = 383, 385, 387 (M+H)⁺
221, 223, 225 (IPM+H)⁺

### β-D-Glucopyranosyl-N,N'-Di-(2-chlorethyl)-phosphorsäurediamid 28β

### Hydrierung von 22β nach Vorschrift C

| | | C | H | N |
|---|---|---|---|---|
| Analyse C₁₀H₂₁Cl₂N₂O₇P (383.10) | ber. | 31.35 | 5.53 | 7.31 |
| | gef. | 31.03 | 5.04 | 6.82 |

### α-D-Galactopyranosyl-N,N'-Di-(2-chlorethyl)-phosphorsäurediamid 29α

### Hydrierung von 23α nach Vorschrift C

- ¹H-NMR:: 500 MHz, D₂O, Nr. 13945
δ = 3.26-3.32 (m, 4H, 2× -CH₂-), 3.635-3.665 (m, 4H, 2 -CH₂-), 3.74-4.05 (m, 5H), 4.098 (m, 1H), 5.642 (dd, 1H, H-1, J_{1,2}=3.1 Hz, J_{1,P}=7.8 Hz).
- FAB-MS:: Nr. MSN 13612
- positiv m/e =: 383 (M+H)⁺
221, 223, 225 (IPM+H)⁺
- negativ m/e =: 381, 383, 385 (M-H)⁻
219, 221, 223 (IPM-H)⁻

### β-D-Galactopyranosyl-N,N'-Di-(2-chlorethyl)-phosphorsäurediamid 29β

### Hydrierung von 23β nach Vorschrift C

- ¹H-NMR:: 500 MHz, D₂O, Nr. 13946
δ = 3.26-3.32 (m, 4H, 2× -CH₂-), 3.605 (d, 1H), 3.63-3.67 (m, 4H, 2× -CH₂-), 3.692 (dd, 1H, J=3.5 und J=10.0 Hz), 3.70-3.95 (m, 4H), 4.950 (t, 1H, H-1, J_{1,2}=J_{1,P}=8.0 Hz).
- FAB-MS:: Nr. MSN 13613
- negativ m/e =: 381, 383, 385 (M-H)⁻
219, 221, 223 (IPM-H)⁻

### α-D-Mannopyranosyl-N,N'-Di-(2-chlorethyl)-phosphorsäurediamid 30α

### Hydrierung von 24α nach Vorschrift C

- ¹H-NMR:: 500 MHz, D₂O, Nr. 13947
δ = 3.26-3.32 (m, 4H, 2× -CH₂-), 3.5-4.0 (m, 9H mit 4H bei 3.63-3.67, 2× -CH₂-), 4.018 (dd, 1H), 5.564 (dd, 1H, H-1, J_{1,2}=2.0 Hz, J_{1,P}=8.0 Hz).
- FAB -MS:: Nr. 13614
- negativ m/e =: 381, 383, 385 (M-H)⁻
219, 221, 223 (IPM-H)⁻

### β-D-Mannopyranosyl-N,N'-di-(2-chlorethyl)-phosphorsäurediamid 30β

### Hydrierung von 24β nach Vorschrift C

- ¹H-NMR:: 500 MHz, D₂O, Nr. 13948
δ = 3.26-3.33 (m, 4H, 2× -CH₂-), 3.45 (m, 1H, H-5), 3.604 (t, 1H, H-4, J_{3,4}=J_{4,5}=9.8 Hz), 3.63-3.67 (m, 4H, 2× -CH₂-), 3.703 (dd, 1H, H-3, J_{2,3}=3.2 Hz, J_{3,4}=9.8 Hz), 3.754 (dd, 1H, H-6a, J_{5,6a}=6.2 Hz, J_{6a,6b}∼12.5 Hz), 3.931 (dd, 1H, H-6b, J_{5,6b}=2.1 Hz, J_{6a,6b}∼12.5 Hz), 4.052 (dd, 1H, H-2, J_{1,2}∼1.1 Hz, J_{2,3}=3.2 Hz), 5.282 (dd, 1H, H-1, J_{1,2}∼1.1 Hz, J_{1,P}=8,6 Hz).
- FAB-MS:: Nr. 13615
- negativ m/e =: 381, 383, 385 (M-H)⁻
219, 221, 223 (IPM-H)⁻

### Di-(2-chlorethyl)-phosphorsäureamid-dichlorid 33 (nach Friedman und Seligman, 1954) J.Am. Chem. Soc. 76, 655

Ansatz: 130 ml POCl₃ (1.4 mol)
50 g Bis-(2-chlorethyl)-amin-hydrochlorid
Ausbeute: 53 g 33 als weiße Kristalle
Fp.: 54 °C (aus Aceton/PE)

| | | C | N | N | Cl |
|---|---|---|---|---|---|
| Analyse C₄H₈Cl₄NOP (258.9) | ber. | 18.56 | 3.11 | 5.41 | 54.77 |
| | gef. | 18.67 | 3.13 | 5.35 | 54.90 |

### Beispiel 2

### Disaccharid-IPM-Konjugate

### Octa-O-acetyl-lactose 35

Eine Mischung aus 100 g Lactose (147 mmol), 400 ml Acetanhydrid und 25 g wasserfreiem Natriumacetat wurde 60 min bei 120-135 °C gerührt. Nach Abkühlen wurde auf Eiswasser gegossen und mit CH₂Cl₂ extrahiert. Die organ. Phase wurde mit ges. NaHCO₃-Lsg. und H₂O neutral gewaschen, über Na₂SO₄ getrocknet und einrotiert. Kristallisation aus Ethariol lieferte 153 g 35 (80% d. Th.).
Fp.: 79-92°C
DC: Kieselgel, Toluol:2-Butanon = 10:4, Rf = 0.43

| | | C | H |
|---|---|---|---|
| Analyse C₂₈H₃₀O₁₉ (678.6) | ber. | 49.56 | 5.64 |
| | gef. | 49.37 | 5.80 |

### Allyl-4-O-(2,3,4,6-tetra-O-acetyl-β-D-galactopyranosyl)-2,3,6,-tri-O-acetyl-D-glucopyranosid 37 (nach Koto et al., 1982) J.Chem. Soc. Jpn. 10, 1651

34 g 35 (50 mmol) wurden in 60 ml CHCl₃ gelöst. Bei 0 °C wurden 20.6 ml Acetylbromid (276 mmol) und 4.56 ml H₂O zugegeben. Nach 2.5 h Rühren bei RT wurde die gelbe klare Lösung bei 30 °C einrotiert und man erhielt einen gelben Schaum (Hepta-O-acetyl-α-D-lactosylbromid) mit
- ¹H-NMR:: 90 MHz, CDCl₃
δ = 1.95-2.20 (m, 21H, 7× -OAc), 3.7-5.65 (m, 13H, Zuckerprotonen), 6.51 (d, 1H, H-1, J_{1,2}=4 Hz).

Der Schaum wurde in 400 ml Allylalkohol bei 35 °C gelöst. Nach Zugabe von 30 g Silbercarbonat wurde 1 d bei RT gerührt (Lichtausschluß), anschließend filtriert und einrotiert. Der Rückstand wurde in Ether aufgenommen und nach erneutem Filtrieren und Einrotieren an Kieselgel 60 (Toluol:2-Butanon = 10:1 → 10:3) chromatographiert. Es wurden 18.2 g 37 (53.8% d. Th.) als klares Öl erhalten.
DC: Kieselgel, Toluol:2-Butanon = 10:4 (10:1), Rf = 0.47 (0.08)

| | | C | H |
|---|---|---|---|
| Analyse C₂₉H₄₀O₁₈ (676.62) | ber. | 51.48 | 5.96 |
| | gef. | 51.44 | 5.92 |

### Allyl-4-O-(2,3,4,6-tetra-O-acetyl-β-D-glucopyranosyl)-2,3,6-tri-O-acetyl-D-glucopyranosid 38 (analog 37)

Ansatz: 29 g α-D-Cellobiose-octaacetat (42.6 mmol) (Ega-Chemie)
17.6 ml Acetylbromid
3.9 ml H₂O
Zwischenprodukt: Hepta-O-acetyl-α-D-cellobiosylbromid
¹H-NMR: 90 MHz, CDCl₃
δ = 6.51 (d, 1H, H-1, J_{1,2}=4 Hz)
Ausbeute: Nach SC (Kieselgel, EE:PE) und Kristallisation aus Diisopropylether wurden 16.5 g 38 (57% d. Th.) erhalten.
Fp.: 179 °C
DC: Kieselgel, Toluol:2-Butanon = 10:4, Rf = 0.49 80,0

| | | C | H |
|---|---|---|---|
| Analyse C₂₉H₄₀O₁₈ (679.62) | ber. | 51.48 | 5.96 |
| | gef. | 51.45 | 6.07 |

### Allyl-4-O-(2,3,4,6-tetra-O-benzyl-β-D-galactopyranosyl)-2,3,6-tri-O-benzyl-D-glucopyranosid 39 (nach Koto et al., 1982) J. Chem. Soc. Jpn. 10, 1651

Ansatz: 19.5 g 37 (28.8 mmol)
800 ml Benzylchlorid
105 g KOH, gepulvert
Ausbeute: Nach SC (Kieselgel, Toluol:2-Butanon = 100:1 → 10:1 und Kristallisation aus EE/Hexan wurden 21.3 g 39 (73% d. Th.) als Nadeln erhalten.
DC: Kieselgel, Toluol:2-Butanon = 10:1, Rf = 0.50
Fp.: 73 °C

| | | C | H |
|---|---|---|---|
| Analyse C₆₄H₆₈O₁₁ (1013.24) | ber. | 75.87 | 6.76 |
| | gef. | 75.66 | 6.63 |

### Allyl-4-O-(2,3,4,6-tetra-O-benzyl-β-D-glucopyranosyl)-2,3,6,-tri-O-benzyl-D-glucopyranosid 40 (analog 39)

Ansatz: 2.65 g 38 (3.92 mmol)
100 ml Benzylchlorid
14.3 g KOH, gepulvert
Ausbeute: Nach SC und Kristallisation aus Diisopropylether/Hexan wurden 2.82 g 40 (71% d. Th.) erhalten.
DC: Kieselgel, Toluol:2-Butanon = 10:1, Rf = 0.50
Fp.: 102 °C

| | | C | H |
|---|---|---|---|
| Analyse C₆₄H₆₈O₁₁ (1013.24) | ber. | 75.87 | 6.76 |
| | gef. | 76.17 | 6.57 |

### 4-O-(2,3,4,6-tetra-O-benzy-β-D-galactopyranosyl)-2,3,4-tri-O-benzyl-D-glucopyranose 43

### A) Isomerisierung mit t - BuOK zum 1 - Propenylether 41

Eine Mischung aus 4.9 g 39 (4.84 mmol) und 1.3 g t-BuOK in 30 ml DMSO wurden unter Stickstoff 2 h bei 110 °C gerührt. DMSO wurde abrotiert, der Rückstand in einem Gemisch Ether/Wasser gelöst. Die Etherphase wurde abgetrennt, die wässrige Phase noch zweimal mit Ether extrahiert. Die vereinigten Etherphasen wurden über Na₂SO₄ getrocknet und einrotiert. Man erhielt 4.02 g 41 (4.13 mmol) als braunes Öl (Rohausbeute 85%):
- DC:: Kieselgel, Toluol:2-Butanon = 10:1, Rf = 0.62

### B) Hydrolyse des 1-Propenylethers 41 mit HgCl₂ zu 43 (nach Gigg und Warren, 1968) J. Chem. Soc. (C), 1903

Zu einer Mischung aus 4.02 g 41 (4.13 mmol) und 1130 mg HgO in 10 ml Aceton/Wasser (10:1) wurden in 5 min 1150 mg HgCl₂ in 10 ml Aceton/Wasser (10:1) zugetropft. Nach 1 h Rühren bei RT wurde durch Celite filtriert, einrotiert und mit Ether versetzt. Die Etherphase wurde mit 10 ml einer halbges. KJ-Lösung und mit Wasser gewaschen. Nach Trocknen über Na₂SO₄ und Einrotiern wurde über Kieselgel chromatographiert (Toluol:2-Butanon = 100:1 → 10:5). Man erhielt 43 als gelbes Öl, das aus Ether/PE kristallisierte: 2.6 g (55% d. Th. bez. auf 39, Anomerengemisch, α:β 2:1 nach ¹³C-NMR).
Fp.: 103 °C
DC: Kieselgel, Toluol:2-Butanon = 10:1, Rf = 0.22

| | | C | H |
|---|---|---|---|
| Analyse C₆₁H₆₄O₁₁ (973.17) | ber. | 75.29 | 6.63 |
| | gef. | 74.79 | 6.65 |

### 4-O-(2,3,4,6-tetra-O-benzyl-β-D-glucopyranosyl)-2,3,4-tri-O-benzyl-D-glucopyranose 44

### 1.) analog 43: Isomerisierung mit t-BuOK zum 1-Propenylether 42 und anschließender Hydrolyse mit HgCl₂

- Ausbeute:: 47.7% d. Th. (nach SC)

### 2.) Isomerisierung mit Tris(triphenylphosphin)rhodiumchlorid ( RhCl(PPh₃)₃) und anschließender Hydrolyse mit 1 N - HCl (nach Corey and Suggs, 1973) J. Org. Chem. 38, 3224

125 mg 40 (0.123 mmol) wurden in 30 ml EtOH/Wasser (10:1) mit 3 mg Diazabicyclo[2.2.2]octan (0.027 mmol) und 12 mg RhCl(PPh₃)₃ (0.009 mmol) 3h gekocht. Dann wurden 6 ml 1 N-HCl zugegeben und nochmal 2 h gekocht. Nach dem Abkühlen wurde mit NaHCO₃-Lsg. versetzt und mit Ether extrahiert. Die organ. Phase wurde mit Wasser gewaschen, über Na₂SO₄ getrocknet und einrotiert. Nach SC (Kieselgel, Toluol:2-Butanon = 100:1 → 100:5 wurden 109 mg 44 (91% d. Th. bez. auf 40, Anomerengemisch, α:β ∼ 3:1 nach ¹³C-NMR) als farbloses Öl erhalten.
- DC:: Kieselgel, Toluol:2-Butanon = 10:1, Rf = 0.22
- ¹H-NMR:: 90 MHz, CDCl₃
δ = 3.05 und 3.25 (2d, 1H, - OH, (α und β ), austauschbar mit D₂O), 3.3-5.2 (m, 28H), 7.1-7.5 (m, 35H, 7× -Ph).
- ¹³C-NMR:: Nr. C14897, CDCl₃
δ = 91.38 (s, C-1α), 97.42 (s, C-1β), 102.68 (s, C-1').
- FAB-MS:: Nr. 13689 (pos., Glycerin, DMF/HCl)
m/e = 973 (M+H)⁺

### 4-O-(2,3,4,6-Tetra-O-benzyl-β-D-galactopyranosyl-2,3,6-tri-O-benzyl-1-O-p-nitrobenzoyl-D-glucopyranose 45

Zu 480 mg 43 (0.49 mmol) in 20 ml CH₂Cl₂ wurden bei RT 2 ml einer Lösung von 130 mg p-Nitrobenzoylchlorid und 0.3 ml Pyridin in CH₂Cl₂ zugetropft. Nach 20h Rühren bei RT war nach DC Kieselgel, Toluol:2-Butanon = 10:1) kaum noch Edukt 43 (Rf = 0.22) und zwei Produkte mit Rf = 0.42 und 0.49. Nach dem Waschen mit 0.5 N-HCl, 1 N-NaHCO₃ und Wasser und Trocknen über Na₂SO₄ wurde ein hochviskoses Öl erhalten. Aus Ethanol kristallisierten 435 mg 45 (79% d.Th., Anomerengemisch, α:β = 3:7 nach ¹H-NMR).
Fp.: 112 °C

| | | C | H | N |
|---|---|---|---|---|
| Analyse C₆₈H₆₇O₁₄N (1122.28) | ber. | 72.77 | 6.02 | 1.25 |
| | gef. | 73.05 | 6.25 | 1.11 |

### 4-O-(2,3,4,6-Tetra-O-benzyl-β-D-glucopyranosyl-2,3,6-tri-O-benzyl-1-O-p-nitrobenzoyl-D-glucopyranose 46 (analog 45)

Ansatz: 520 mg 44 (0.534 mmol)
150 mg p Nitrobenzoylchlorid
0.4 ml Pyridin
Ausbeute: Nach 20 h zeigte DC (Kieselgel, Toluol:2-Butanon = 10:1) zwei Produkte mit Rf = 0.43 und 0.49 neben wenig Edukt mit Rf = 0.22. Nach SC (Kieselgel, Toluol:2-Butanon = 20:1) wurden 320 mg 46 als Öl (53.4% d. Th.) erhalten. Aus Diisopropylether kristallisierte 46α.
Fp.: 169 °C

| | | C | H | N |
|---|---|---|---|---|
| Analyse C₆₈H₆₇O₁₄N (1122.28) | ber. | 72.77 | 6.02 | 1.25 |
| | gef. | 72.70 | 6.01 | 1.07 |

### O-[4-O-(2,3,4,6-Tetra-O-benzyl-β-D-glucopyranosyl)-2,4,6-tri-O-benzyl-α-D-glucopyranosyl]-trichloracetimidat 47 (analog 25α)

- Ansatz:: 130 mg 44 (0.133 mmol)
60 »l Trichloracetonitril
5.3 mg NaH
- Ausbeute:: Nach SC (Kieselgel, E:PE = 2:3) wurden 120 mg 47 (80% d. Th.) als klares farbloses Öl erhalten.
- DC:: Kieselgel, E:PE = 3:2, Rf = 0.49
- ¹H-NMR:: 90 MHz, CDCl₃
δ = 3.3-5.2 (m, 27H), 6.44 (d, 1H, H-1, J_{1,2}=4 Hz), 7.1-7.4 (m, 35H, 7× -Ph), 8.57 (s, 1H, -NH-, austauschbar mit D₂O).

C₆₃H₆₄Cl₃NO₁₁ (1117.56)

### 4-O-(2,3,4,6-Tetra-O-benzyl-β-D-galactopyranosyl)-2,3,6-tri-O-benzyl-D-glucopyranosyl-N,N'-Di-(2-chlorethyl)-phosphorsäurediamid 48

### Nach Vorschrift A

- Ansatz:: 100 mg 45 (0.089 mmol)
21 mg 4b (0.095 mmol)
- Ausbeute:: Nach SC (Kieselgel, EE:PE = 40:60 → 70:30) wurden 2 Fraktionen erhalten:
F1: 7mg 48, β>>α (6.7% d. Th.)
F2: 40 mg 48, α>>β (38.2% d. Th.)
- DC:: Kieselgsl, EE/PE = 80:20, Rf(α) = 0.37, Rf(β) = 0.42

C₆₅H₇₃Cl₂N₂O₁₂P (1176.18)
- ¹H-NMR:: Nr. H15189, 90MHz, CDCl₃, α>>β
Nr. H15326, 90MHz CDCl₃, β>>α
- HPLC, anal.:: Kieselgel, EE:Hexan:MeOH = 72:28:0.7, Flow 1.0 ml/min, Rt(α) = 10.32' Rt(β) = 8.73'
- HPLC, präp.:: Kieselgel, EE:Hexan:MeOH = 64:36:0.5, Flow 10.0 ml/min, Rt(α) = 35' Rt(β) = 27'

### 4-O-(2,3,4,6-Tetra-O-benzyl-β-D-glucopyranosyl)-2,3,6-tri-O-benzyl-D-glucopyranosyl-N,N'-Di-(2-chlorethyl)-phophorsäurediamid 49

Nach Vorschrift B
- Ansatz:: 40 mg 47 (0.036 mmol)
9 mg 4b (0.041 mmol)
- Ausbeute:: Nach SC (Kieselgel, EE:PE = 60:40) und präp. HPLC wurden 2 Fraktionen (jeweils klares, farbloses Öl) erhalten:
F1: 18 mg 49β (42.5% d. Th.)
F2: 7 mg 49α (10.9% d. Th.)
- DC:: Kieselgel, EE/PE = 80:20, Rf(α) = 0.35, Rf(β) = 0.42
C₆₅H₇₃Cl₂N₂O₁₂P (1176.18)
- HPLC, anal.:: Kieselgel, EE:Hexan:MeOH = 72:28:1, Flow 1.0 ml/min Rt(α) = 7.03', Rt(β) = 5.89'
- HPLC, präp.:: Kieselgel, EE:Hexan:MeOH = 64:36:0.5, Flow 10.0 ml/min Rt(α) = 35', Rt(β) = 25'

### 4-O-(β-D-galactopyranosyl)-α-D-glucopyranosyl-N,N'-Di-(2-chlorethyl)-phosphorsäurediamid 50α

### Hydriervng von 48α nach Vorschrift C

C₁₆H₃₁Cl₂N₂O₁₂P (541.31)
- ¹H-NMR:: 500 MHz, D₂O, Nr. H15701
δ = 3.27-3.34 (m, 4H, 2× -CH₂-), 3.563 (dd, 1H, H-2', J_{1',2'}=8.0 Hz, J_{2',3'}=9.8 Hz), 3.65-4.0 (m, 15H mit 4H bei 3.65-3.68, 2× -CH₂-), 4.478 (d, 1H, H-1', J_{1',2'}=8.0 Hz), 5.623 (dd, 1H, H-1, J_{1,2}=3.6 Hz, J_{1,P}=7.8 Hz).
- FAB-MS:: Nr. MSN 14075
- positiv m/e =: 221, 223, 225 (IPM+H)⁺
545, 547, 549 (M+H)⁺

### 4-O-(β-D-galactopyranosyl)-β-D-glucopyranosyl-N,N'-Di-(2-chlorethyl)-phosphorsäurediamid 50β

### Hydrierung von 48β nach Vorschrift C

C₁₆H₃₁Cl₂N₂O₁₂P (541.31)
- ¹H-NMR:: 500 MHz, D₂O, ¹H-¹H-2D-COSY, Nr. 15834
δ = 3.27-3.34 (m, 4H, 2× -CH₂-), 3.43 (dd, 1H, H-2, J_{1,2}=8.0 Hz), 3.558 (dd, 1H, H-2', J_{1',2'}=8.0 Hz, J_{2',3'}=10.0 Hz), 3.65-3.68 (m, 4H, 2× -CH₂-), 3.68-3.90 (m, 8H), 3.938 (dd, 1H, J=3.4 und J∼1.0 Hz), 3.989 (dd, 1H, J=1.9 und J=12.6 Hz), 4.473 (d, 1H, H- 1', J_{1',2'}=8.0 Hz), 5.049 (t, 1H, H-1, J_{1,2}=J_{1,P}=8.0 Hz).
- FAB-MS:: Nr. MSN 14076
- positiv m/e =: 221, 223, 225 (IPM+H)⁺
545, 547, 549 (M+H)⁺

### 4-O-(β-D-glucopyranosyl)-α-D-glucopyranosyl-N,N'-Di-(2-chlorethyl)-phosphorsäurediamid 51α

### Hydrierung von 49α nach Vorschrift C

C₁₆H₃₁Cl₂N₂O₁₂P (541.31)
- ¹H-NMR:: 500 MHz, D₂O, ¹H-¹H-2D-COSY, Nr. 15856
δ = 3.27-3.32 (m, 4H, 2× -CH_{1,2}-), 3.333 (dd, 1H, H-2', J_{1',2'}=7.9 Hz, J_{2',3'}=9.4 Hz), 3.40-3.45 (m, 2H), 3.47-3.55 (m, 2H), 3.64-3.68 (m, 4H, 2× -CH₂-), 3.69 (dd, 1H, H-2), 3.70-3.99 (m, 6H), 4.537 (d, 1H, H-1', J_{1',2'}=7.9 Hz), 5.622 (dd, 1H, H-1, J_{1,2}=3.6 Hz, J_{1,P}=7.8 Hz).
- FAB-MS:: Nr. MSN 14077
- positiv m/e =: 221, 223, 225 (lPM+H)⁺
545, 547 (M+H)⁺

### 4-O-(β-D-glucopyranosyl)-β-D-glucopyranosyl-N,N'-Di-(2-chlorethyl)-phosphorsäurediamid 51β

### Hydrierung von 49β nach Vorschrift C

C₁₆H₃₁Cl₂N₂O₁₂P (541.31)
- ¹H-NMR:: 500 MHz, D₂O, ¹H-¹H-2D-COSY, Nr. 15857
δ = 3.27-3.32 (m, 4H, 2× -CH_{1,2}-), 3.33 (dd, 1H, H-2', J_{1',2'}=8.0 Hz, J_{2',3'}∼10 Hz), 3.428 (dd, 1H, H-2, J_{1,2}=8.0 Hz, J_{2,3}=10.0 Hz), 3.44-3.54 (m, 3H), 3.64-3.68 (m, 4H, 2× -CH₂-), 3.69-3.72 (m, 3H), 3.745 (dd, 1H, H-6'a, J_{5',6'a}=5.8 Hz, J_{6'a,6'b}=12.3 Hz), 3.855 (m, 1H, H-6b), 3.928 (dd, 1H, H-6'b, J_{5',6'b}=2.1 Hz, J_{6'a,6'b}∼12.3 Hz), 3.990 (dd, 1H, H-6a, J_{5,6a}=2.0 Hz, J_{6a,6b}=12.2 Hz), 4.532 (d, 1H, H-1', J_{1',2'}=8.0 Hz), 5.044 (t, 1H, H-1, J_{1,2}=J_{1,P}=8.0 Hz).
- FAB - MS:: Nr. MSN 14078
- positiv m/e =: 221, 223, 225 (IPM+H)⁺
545, 547, 549 (M+H)⁺

### Beispiel 3

Maltotriose wird peracetyliert (Natriumacetat/Acetanhydrid), (R_{f} 0,48, CHCl₃/Essigester 1:1, Kieselgel), daraus mit HBr/Eisessig bei 0°C das 1-Bromid (R_{f} = 0,58, gleiche Bedingungen wie oben); daraus mit Allylalkohol/Ag₂CO₃ das 1-Alkyl-maltotrisid (R_{f} = 0,60, gl. Bedingungen wie oben). Mit Benzylchlorid/KOH bei 120°C daraus die Alkyl-2,3,6,2',3',6',2'',3'',4'',6'' deca-O-benzyl-maltotriosid (R_{f}= 0,51, Toluo1lEssigester 10:1, Kieselgel). Nach Isomerisierung zum Enolether Versei£ung zur 1-OH Verbindung mit 1N HCl (R_{f}=0,17, Toluol/Essigester 10:1, Kieselgel). Daraus das Trichloracetimidat durch Umsetzung mit NaH und Trichloracetonitril (R_{fα} =0.48, gleiche Bedingungen wie oben). Daraus in Acetonitril mit Ifosfamidmustard unter Rückfluß das Glykoconjugat (R_{f}=0,24, Essigester/Hexan 6:4, Kieselgel). Durch Hydrierung mit 10%igem Pd/Aktivkohle in CH₃OH bei Raumtemperatur Abspaltung der Benzylgruppen (R_{f}= 0,22, CHCl₃/Methanol 1:1, Kieselgel).

## Patentansprüche (Patentansprüche für folgende(n) Vertragsstaat(en): AT, BE, CH, DE, FR, GB, IT, LI, LU, NL, SE)

1. Glycokonjugate von Ifosfamid mustard der Formel:
α-D-Glucopyranosyl-N,N'-di(2-chloroethyl)-phosphorsäurediamid,
β-D-Glucopyranosyl-N,N'-di-(2-chlorethyl)-phosphorsäurediamid,
α-D-Galactopyranosyl-N,N'-di-(2-chlorethyl)-phorphorsäurediamid,
α-D-Mannopyranosyl-N,N'-di-(2-chlorethyl)-phosphorsäurediamid,
β-D-Mannopyranosyl-N,N'-di-(2-chlorethyl)-phosphorsäurediamid,
4-O-(β-D-Galactopyranosyl)-alpha-D-glucopyranosyl-N,N'-di(2-chlorethyl)-phosphorsäurediamid,
4-O-(β-D-Galactopyranosyl)-N,N'-di-(2-chlorethyl)-phosphorsäurediamid,
4-O-(β-D-Galactopyranosyl)-β-D-glucopyranosyl-N,N'-di(2-chlorethyl)-phosphorsäurediamid,
4-O(β-D-Glucopyranosyl)-alpha-D-glucopyranosyl-N,N'-di-(2-chlorethyl)-phosphorsäurediamid,
4-O-(β-D-Glucopyranosyl)-β-D-glucopyranosyl-N,N'-di-(2-chlorethyl)-phosphorsäurediamid.

2. Verfahren zur Herstellung von Glycokonjugaten von Phosphoramid mustard und Ifosfamid mustard der allgemeinen Formel 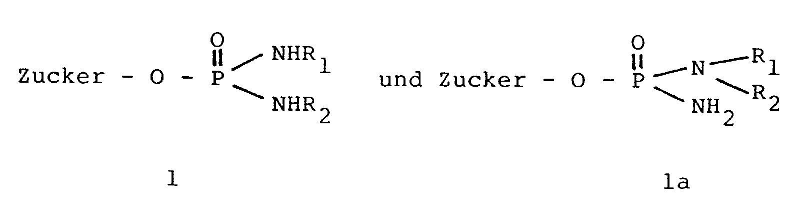 wobei die Verknüpfung des Zuckers mit der Phosphoramid-Lost-Funktion bzw. der Ifosfamid-Lost-Funktion vorzugsweise in der 1-Stellung erfolgt und R₁ und R₂ gleich oder verschieden sein und Wasserstoff, niederes C₁-C₄-Alkyl, C₁-C₆-Halogenalkyl, vorzugsweise C₁-C₄-Halogenalkyl und insbesondere C₂-Halogenalkyl bedeuten können und als Zucker Mono-, Di- oder Polysaccharide in allen existierenden isomeren und enantiomeren Formen vorliegen können, dadurch gekennzeichnet, daß man in an sich bekannter Weise geschützte Bromzucker, insbesondere benzylgeschützte Bromzucker mit den entsprechenden Phosphorverbindungen konjugiert und die erhaltene Verbindung von den Schutzgruppen befreit.

3. Verbindung nach Anspruch 1 oder 2 zur Verwendung als Antitumormittel.

4. Verbindungen nach Anspruch 3 zur Verwendung gegen Mammacarcinom, Morbus Hodgkin oder Tumoren im Magen- und Darmbereich.

## Patentansprüche (Patentansprüche für folgende(n) Vertragsstaat(en): ES, GR)

1. Verfahren zur Herstellung von Glycokonjugaten von Phosphoramid mustard und Ifosfamid mustard der allgemeinen Formel 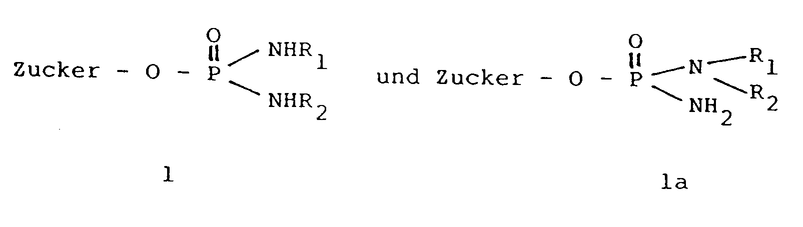 wobei die Verknüpfung des Zuckers mit der Phosphoramid-Lost-Funktion bzw. der Ifosfamid-Lost-Funktion vorzugsweise in der 1-Stellung erfolgt und R₁ und R₂ gleich oder verschieden sein und Wasserstoff, niederes C₁-C₄-Alkyl, C₁-C₆-Halogenalkyl, vorzugsweise C₁-C₄-Halogenalkyl und insbesondere C₂-Halogenalkyl bedeuten können und als Zucker Mono-, Di- oder Polysaccharide in allen existierenden isomeren und enantiomeren Formen vorliegen können, dadurch gekennzeichnet, daß man in an sich bekannter Weise geschützte Bromzucker, insbesondere benzylgeschützte Bromzucker mit den entsprechenden Phosphorverbindungen konjugiert und die erhaltene Verbindung von den Schutzgruppen befreit.

2. Verwendung der nach Anspruch 1 erhaltenen Verbindungen zur Herstellung von Antitumormitteln.

3. Verwendung der Verbindung nach Anspruch 2 zur Herstellung von Mitteln gegen Mammacarcinom, Morbus Hodgkin oder Tumoren im Magen- und Darmbereich.

## Claims (Claims for the following Contracting State(s): AT, BE, CH, DE, FR, GB, IT, LI, LU, NL, SE)

1. Glycoconjugates of ifosfamide mustard with the formula:
α-D-glucopyranosyl N,N'-di(2-chloroethyl) phosphoric acid diamide,
β-D-glucopyranosyl N,N'-di(2-chloroethyl) phosphoric acid diamide,
α-D-galactopyranosyl N,N'-di(2-chloroethyl) phosphoric acid diamide,
α-D-mannopyranosyl N,N'-di(2-chloroethyl) phosphoric acid diamide,
β-D-mannopyranosyl N,N'-di(2-chloroethyl) phosphoric acid diamide,
4-O-(β-D-galactopyranosyl) α-D-glucopyranosyl N,N'-di(2-chloroethyl) phosphoric acid diamide,
4-O-(β-D-galactopyranosyl) N,N'-di(2-chloroethyl) phosphoric acid diamide,
4-O-(β-D-galactopyranosyl) β-D-glucopyranosyl N,N'-di(2-chloroethyl) phosphoric acid diamide,
4-O-(β-glucopyranosyl) α-D-glucopyranosyl N,N'-di(2-chloroethyl) phosphoric acid diamide,
4-O-(β-glucopyranosyl) β-D-glucopyranosyl N,N'-di(2-chloroethyl) phosphoric acid diamide.

2. Method for the preparation of glycoconjugates of phosphoric acid amide mustard and ifosfamide mustard with the general formula: 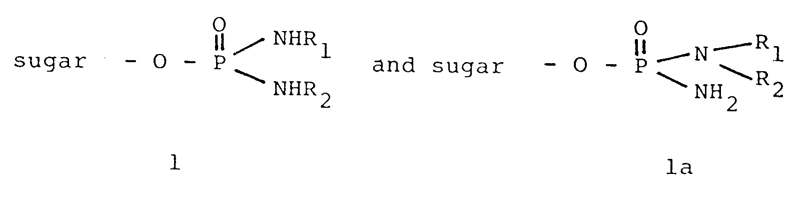 where the sugar is linked to the phosphoric acid amide-lost residue, and the ifosfamide-lost residue, resp., preferably in the 1-position, and where R₁ and R₂, which can be the same or different, denote hydrogen, lower C₁-C₄ alkyl , C₁-C₆ halogenoalkyl, preferably C₁-C₄ halogenoalkyl, and particularily C₂-halogenoalkyl, and where as sugar there can be present mono-, di-, or polysaccharides in all existing isomeric and enantiomeric forms, characterized by conjugating in a known way protected brominated sugars, particularly benzyl-protected brominated sugars, with the respective phosphorus compounds, and freeing the resulting compound from the protective groups.

3. The compound according to claim 1 or 2 as anti-tumour agents.

4. The compounds according to claim 3 for use against breast carcinoma, Morbus Hodgkin, or tumours in the gastrointestinal tract.

## Claims (Claims for the following Contracting State(s): ES, GR)

1. Method for the preparation of glycoconjugates of phosphoric acid amide mustard and ifosfamide mustard with the general formula: 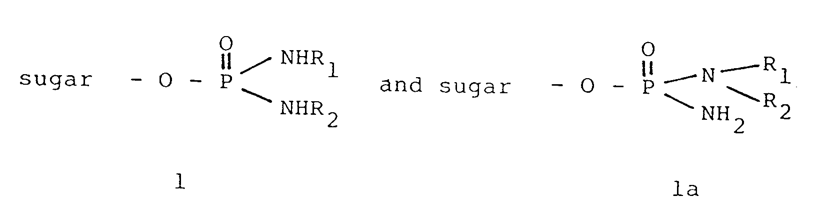 where the sugar is linked to the phosphoric acid amide-lost residue, and the ifosfamide-lost residue, resp., preferably in the 1-position, and where R₁ and R₂, which can be the same or different, denote hydrogen, lower C₁-C₄ alkyl , C₁-C₆ halogenoalkyl, preferably C₁-C₄ halogenoalkyl, and particularily C₂-halogenoalkyl, and where as sugar there can be present mono-, di-, or polysaccharides in all existing isomeric and enantiomeric forms, characterized by conjugating in a known way protected brominated sugars, particularly benzyl-protected brominated sugars, with the respective phosphorus compounds, and freeing the resulting compound from the protective groups.

2. Use of the compounds obtained by the method according to claim 1 for the preparation of antitumour agents.

3. Use of the compounds according to claim 2 for the preparation of means against breast carcinoma, Morbus Hodgkin, or tumours in the gastrointestinal tract.

## Revendications (Revendications pour l'(les) Etat(s) contractant(s) suivant(s): AT, BE, CH, DE, FR, GB, IT, LI, LU, NL, SE)

1. Glycoconjugués de moutarde ifosfamidique de formule :
α-D-glucopyranosyl-N,N'-di(2-chloréthyl)phosphodiamide,
β-D-glucopyranosyl-N,N'-di(2-chloréthyl)phosphodiamide,
α-D-galactopyranosyl-N,N'-di(2-chloréthyl)phosphodiamide,
α-D-mannopyranosyl-N,N'-di(2-chloréthyl)phosphodiamide,
β-D-mannopyranosyl-N,N'-di(2-chloréthyl)phosphodiamide,
4-O-(β-D-galactopyranosyl)-alpha-D-glucopyranosyl-N,N'-di(2-chloréthyl)phosphodiamide,
4-O-(β-D-galactopyranosyl)-N,N'-di(2-chloréthyl)phosphodiamide,
4-O-(β-D-galactopyranosyl)-β-D-glucopyranosyl-N,N'-di(2-chloréthyl)phosphodiamide,
4-O-(β-D-glucopyranosyl)-alpha-D-glucopyranosyl-N,N'-di(2-chloréthyl)phosphodiamide,
4-O-(β-D-glucopyranosyl)-β-D-glucopyranosyl-N,N'-di(2-chloréthyl)phosphodiamide.

2. Procédé de préparation de glycoconjugués de moutarde phosphoramidique et de moutarde ifosfamidique répondant à la formule générale : 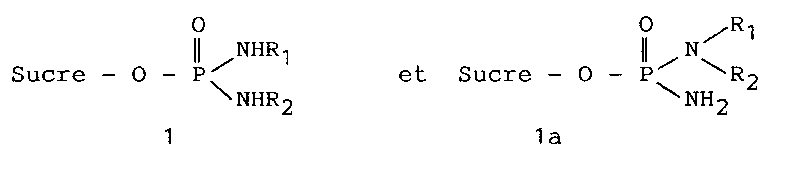 où la liaison du sucre avec la fonction moutarde phosphoramidiqu ou la fonction moutarde ifosfamidique apparaît, de préférence, à la position 1, et R₁ et R₂ sont identiques ou différents et peuvent signifier l'hydrogène, un groupe alkyle inférieur en C₁-C₄, un groupe halogénoalkyle en C₁-C₆, de préférence un groupe halogénoalkyle en C₁-C₄, et en particulier un groupe halogénoalkyle en C₂, et les sucres présents peuvent être des mono-, di- ou polysaccharides sous toutes leurs formes isomères et énantiomères existantes, caractérisé en ce qu'on conjugue, d une manière connue en soi, des bromosucres protégés, en particulier des bromo-sucres protégés avec un groupe benzyle, avec les composés phosphorés correspondants, et on débarrasse le composé obtenu des groupes protecteurs.

3. Composé selon la revendication 1 ou 2 destiné à être utilisé comme un agent antitumoral.

4. Composés selon la revendication 3 destinés à être utilisés contre le cancer du sein, la maladie de Hodgkin ou des tumeurs de l'appareil gastro-intestinal.

## Revendications (Revendications pour l'(les) Etat(s) contractant(s) suivant(s): ES, GR)

1. Procédé de préparation de glycoconjugués de moutarde phosphoramidique et de moutarde ifosfamidique répondant à la formule générale : 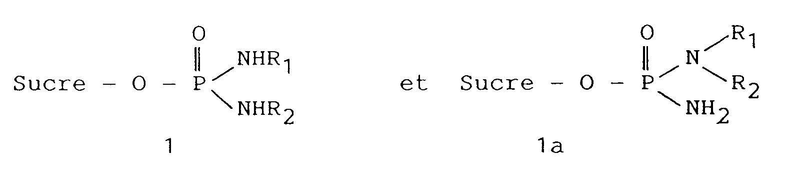 où la liaison du sucre avec la fonction moutarde phosphoramidiqu ou la fonction moutarde ifosfamidique apparaît, de préférence, à la position 1, et R₁ et R₂ sont identiques ou différents et peuvent signifier l'hydrogène, un groupe alkyle inférieur en C₁-C₄, un groupe halogénoalkyle en C₁-C₆, de préférence un groupe halogénoalkyle en C₁-C₄, et en particulier un groupe halogénoalkyle en C₂, et les sucres présents peuvent être des mono-, di- ou polysaccharides sous toutes leurs formes isomères et énantiomères existantes, caractérisé en ce qu'on conjugue, d'une manière connue en soi, des bromosucres protégés, en particulier des bromo-sucres protégés avec un groupe benzyle, avec les composés phosphorés correspondants, et on débarrasse le composé obtenu des groupes protecteurs.

2. Utilisation des composés obtenus selon la méthode exposée dans la revendication 1, destinés à la préparation d'agents antitumoraux.

3. Utilisation des composés selon la revendication 2 destinés à la préparation d'agents contre le cancer du sein , la maladie de Hodgkin ou des tumeurs de l'apparaeil gastro-intestinal.
